(19) Europäisches Patentamt / European Patent Office / Office européen des brevets

(11) **EP 3 074 554 B1**

(12) **EUROPEAN PATENT SPECIFICATION**

(45) Date of publication and mention
of the grant of the patent:
**19.05.2021 Bulletin 2021/20**

(51) Int Cl.:
***C12N 15/10*** *(2006.01)*     ***C40B 40/08*** *(2006.01)*
***C40B 50/00*** *(2006.01)*

(21) Application number: **14866712.4**

(22) Date of filing: **25.11.2014**

(86) International application number:
**PCT/US2014/067444**

(87) International publication number:
**WO 2015/081114 (04.06.2015 Gazette 2015/22)**

(54) **LIBRARIES OF NUCLEIC ACIDS AND METHODS FOR MAKING THE SAME**

NUKLEINSÄUREBIBLIOTHEKEN UND VERFAHREN ZUR HERSTELLUNG DAVON

BANQUES D'ACIDES NUCLÉIQUES ET PROCÉDÉS POUR LES PRÉPARER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2013 US 201361909537 P**

(43) Date of publication of application:
**05.10.2016 Bulletin 2016/40**

(60) Divisional application:
**21168127.5**

(73) Proprietor: **Gen9, Inc.**
**Boston MA 02210 (US)**

(72) Inventors:
• **JACOBSON, Joseph**
  **Newton,**
  **Massachusetts 02459 (US)**
• **SCHINDLER, Daniel**
  **Newton,**
  **Massachusetts 02464 (US)**
• **SAAEM, Ishtiaq**
  **Chaka 1213 (BD)**
• **GUIDO, Nicholas James**
  **Milford,**
  **Massachusetts 01757 (US)**

(74) Representative: **Mewburn Ellis LLP**
**Aurora Building**
**Counterslip**
**Bristol BS1 6BX (GB)**

(56) References cited:
**WO-A1-2012/084923     KR-B1- 100 491 810**
**US-A1- 2002 132 308     US-A1- 2008 287 320**
**US-A1- 2013 059 296**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> Gen9, Inc.

<120> LIBRARIES OF NUCLEIC ACIDS AND METHODS FOR MAKING THE SAME

<130> 127662-014601/PCT

<140> Not yet assigned
<141> 2014-11-25

<150> US 61/909,537
<151> 2013-11-27

<160> 27

<170> PatentIn version 3.5

<210> 1
<211> 14
<212> DNA
<213> Artificial Sequence

<220>
<223> Synthetic construct

<400> 1
tccgatttac gggt          14

<210> 2
<211> 14
<212> DNA
<213> Artificial Sequence

<220>
<223> Synthetic construct

<400> 2
tccgatctac gggt          14

<210> 3
<211> 14
<212> DNA
<213> Artificial Sequence

<220>
<223> Synthetic construct

<400> 3
tcttgtccga ttta        14

<210> 4
<211> 14
<212> DNA
<213> Artificial Sequence

<220>
<223> Synthetic construct

<400> 4
tcttgtccga tcta        14

<210> 5
<211> 14
<212> DNA
<213> Artificial Sequence

<220>
<223> Synthetic construct

<400> 5
agccgctggg ttta        14

<210> 6
<211> 14
<212> DNA
<213> Artificial Sequence

<220>
<223> Synthetic construct

<400> 6
cgggtagccg ctgg        14

<210> 7
<211> 14
<212> DNA
<213> Artificial Sequence

<220>
<223> Syntehtic construct

<400> 7
taaatcggac aaga        14

<210> 8
<211> 14
<212> DNA
<213> Artificial Sequence

<220>
<223> Synthetic construct

<400> 8
tagatcggac aaga        14

<210> 9
<211> 14
<212> DNA
<213> Artificial Sequence

<220>
<223> Synthetic construct

<400> 9
acccgtaaat cgga        14

<210> 10
<211> 14
<212> DNA
<213> Artificial Sequence

<220>
<223> Synthetic construct

<400> 10
acccgtagat cgga        14

<210> 11
<211> 14
<212> DNA
<213> Artificial Sequence

<220>
<223> Synthetic construct

<400> 11
ccagcggcta cccg        14

<210> 12
<211> 14
<212> DNA
<213> Artificial Sequence

<220>
<223> Synthetic construct

<400> 12
taaacccagc ggct        14

<210> 13
<211> 28
<212> DNA
<213> Artificial Sequence

<220>
<223> Synthetic construct

<400> 13
tccgatttac gggtagccgc tgggttta        28

<210> 14
<211> 28
<212> DNA
<213> Artificial Sequence

<220>
<223> Synthetic construct

<400> 14
ccagcggcta cccgtaaatc ggacaaga        28

<210> 15
<211> 28
<212> DNA
<213> Artificial Sequence

<220>
<223> Synthetic construct

<400> 15
tccgatctac gggtagccgc tgggttta        28

<210> 16
<211> 28
<212> DNA
<213> Artificial Sequence

<220>
<223> Synthetic construct

<400> 16
ccagcggcta cccgtagatc ggacaaga        28

<210> 17
<211> 67
<212> DNA
<213> Artificial Sequence

<220>
<223> Synthetic construct

<400> 17

```
atcgctcagg atttcttgga agaagaagct aagcacgctg ttcaaatgtt gttgccagaa        60

aacgtta                                                                  67
```

<210> 18
<211> 64
<212> DNA
<213> Artificial Sequence

<220>
<223> Synthetic construct

<400> 18

atcgctcagg atttggaacc ggaagctaag cacgctgttc aaatgttgtt gccagaaaac     60

gtta     64

<210> 19
<211> 70
<212> DNA
<213> Artificial Sequence

<220>
<223> Synthetic construct

<400> 19

atcgctcagg atctgttgga accggaagct aagcacgctg ttcaaccgat gttgttgcca     60

gaaaacgtta     70

<210> 20
<211> 69
<212> DNA
<213> Artificial Sequence

<220>
<223> Synthetic construct

<400> 20

gactctaagg aagactcttt gatcgctacc ggttctcacc accaccacca ccaccaccag     60

tctggtgac     69

<210> 21
<211> 68
<212> DNA
<213> Artificial Sequence

<220>
<223> Synthetic construct

<400> 21

gactctaagg aagactcttt gatcgctacc ggttctcacc accaccacca ccaccacagt     60

ctggtgac     68

<210> 22
<211> 70
<212> DNA
<213> Artificial Sequence

<220>
<223> Synthetic construct

<400> 22

gactctaagg aagactcttt gatcgctacc ggtatctcac caccaccacc accaccacca        60

gtctggtgac        70

<210> 23
<211> 56
<212> DNA
<213> Artificial Sequence

<220>
<223> Synthetic construct

<400> 23
accaattgtc tcactacaga cacggtaccc tgacagaaga tacaattgtt gttctt        56

<210> 24
<211> 44
<212> DNA
<213> Artificial Sequence

<220>
<223> Synthetic construct

<400> 24
accaattgtc tcactacctg acagaagata caattgttgt tctt 44

<210> 25
<211> 68
<212> DNA
<213> Artificial Sequence

<220>
<223> Synthetic construct

<400> 25

accaattgtc tcactacaga cacggtaccc tgacagaaga tacaacatga ccgaagcttg        60

ttgttctt        68

<210> 26
<211> 180
<212> DNA
<213> Artificial Sequence

<220>
<223> Synthetic construct

<400> 26

atcgctcagg atttcttgga agaagaagct aagcacgctg ttcaaatgtt gttgccagaa        60

aacgttagac tctaaggaag actctttgat cgctaccggt tctcaccacc accaccacca        120

ccaccagtct ggtgacacca attgtctcac tacctgacag aagatacaat tgttgttctt        180

<210> 27
<211> 175
<212> DNA
<213> Artificial Sequence

<220>
<223> Synthetic construct

<400> 27

```
atcgctcagg atttggaacc ggaagctaag cacgctgttc aaatgttgtt gccagaaaac        60

gttagactct aaggaagact ctttgatcgc taccggttct caccaccacc accaccacca       120

cagtctgtga caccaattgt ctcactacct gacagaagat acaattgttg ttctt           175
```

**Description**

**FIELD OF THE INVENTION**

Methods and compositions of the invention relate to nucleic acid libraries, and particularly to the design and assembly of nucleic acid libraries containing non-random variants.

**BACKGROUND**

Recombinant and synthetic nucleic acids have many applications in research, industry, agriculture, and medicine. Recombinant and synthetic nucleic acids can be used to express and obtain large amounts of polypeptides, including enzymes, antibodies, growth factors, receptors, and other polypeptides that may be used for a variety of medical, industrial, or agricultural purposes. Recombinant and synthetic nucleic acids also can be used to produce genetically modified organisms including modified bacteria, yeast, mammals, plants, and other organisms. Genetically modified organisms may be used in research (e.g., as animal models of disease, as tools for understanding biological processes, etc.), in industry (e.g., as host organisms for protein expression, as bioreactors for generating industrial products, as tools for environmental remediation, for isolating or modifying natural compounds with industrial applications, etc.), in agriculture (e.g., modified crops with increased yield or increased resistance to disease or environmental stress, etc.), and for other applications. Recombinant and synthetic nucleic acids also may be used as therapeutic compositions (e.g., for modifying gene expression, for gene therapy, etc.) or as diagnostic tools (e.g., as probes for disease conditions, etc.).

Numerous techniques have been developed for modifying existing nucleic acids (e.g., naturally occurring nucleic acids) to generate recombinant nucleic acids and nucleic acid variants. In particular, variant libraries have been used to select or screen nucleic acids or proteins products that have a desired property. As such, there is significant need in the de novo synthesis of nucleic acids for a wide range of applications.

**SUMMARY OF THE INVENTION**

Disclosure herein relates to methods of producing non-random nucleic acid libraries comprising a plurality of pre-selected or predetermined sequences of interest. Other disclosure herein relates to non-random nucleic acid libraries comprising a plurality of pre-selected or predetermined sequences of interest. An aspect of the invention provides a method for generating a nucleic acid library as set out in claim 1.

Disclosure herein relates to methods for producing non-random nucleic acid libraries comprising the steps of (a) providing a first plurality of partial double-stranded nucleic acids in a first volume, wherein each of the first plurality of double-stranded nucleic acids has identical single-stranded overhangs, wherein each of the first plurality of partial double-stranded nucleic acids has a predetermined sequence different than another predetermined sequence in the first plurality of partial double-stranded nucleic acids; (b) providing a second plurality of partial double-stranded nucleic acids in a second volume, wherein each of the second plurality of partial double-stranded nucleic acids has identical single-stranded overhangs that are complementary to the overhangs in the first plurality of partial double-stranded nucleic acids, and (c) assembling the library of nucleic acids by mixing the first plurality of partial double-stranded nucleic acids with the second plurality of partial double-stranded nucleic acids under conditions to hybridize the complementary overhangs to form the library of non-random variant target nucleic acids. In some embodiments, the second plurality of partial double-stranded nucleic acids has a predetermined sequence that can be different than another sequence in the second plurality of partial double-stranded nucleic acids. Yet in other embodiments, the second plurality of partial double-stranded nucleic acids has a predetermined sequence that can be the same as another sequence in the second plurality of partial double-stranded nucleic acids

In some embodiments, the first and the second pluralities of partial double-stranded nucleic acids have 3' overhangs. Yet in other embodiments, the first and the second pluralities of partial double-stranded nucleic acids have 5' overhangs.

In some embodiments, the step of assembling can be performed in a single reaction volume.

In some embodiments, in the step of assembling, the complementary overhangs hybridize to form gapless junctions. In some embodiments, the gapless junctions are ligated.

In some embodiments, the method comprises providing a first plurality of sets of blunt-ended double-stranded nucleic acids in the first volume, wherein a first nucleic acid of a first set of blunt-ended double stranded nucleic acids has a sequence that is offset by n bases from a second nucleic acid of the first set of blunt-ended double stranded nucleic acids, and wherein each double-stranded nucleic acid in each set of blunt-ended double-stranded nucleic acids is a variant of another double-stranded nucleic acid in the set. In some embodiments, the method further comprises providing a second plurality of sets of blunt-ended double stranded nucleic acids in the second volume, wherein a first nucleic acid of the second set of blunt-ended double-stranded nucleic acids has a sequence that is offset by n bases from a second nucleic acid of the second set of blunt-ended double-stranded nucleic acids. In some embodiments, n can be 2, 3, 4, 5, 6, 7, or 8 bases. In some embodiments, n can be greater than 8 bases. For example, n can be 9, 10, 11, 12, 13, 14, 15, 16, 17, 18, 19, 20 or more bases. The first plurality of sets of blunt-ended double stranded nucleic acids can be melted or de-hybridized in the first volume to form single-stranded nucleic acids in the first volume. Similarly, the second plurality of sets of blunt-ended double stranded nucleic acids in the second volume can be denatured or dehybridized to form single-stranded nucleic acids in the second volume. The plurality of single-stranded oligonucleotides can anneal to form the first plurality of partial double-stranded oligonucleotides having single-stranded overhangs in the first volume and the second plurality of partial double-stranded oligonucleotides having single-stranded overhangs in the second volume.

In some embodiments, each double-stranded nucleic acid in the second plurality of sets of blunt-ended double-stranded nucleic acids is a variant of another double-stranded nucleic acid in the set.

In some embodiments, the method can further comprise a third plurality of partial double-stranded nucleic acids in a third volume, wherein each of the third plurality of double-stranded nucleic acids has identical single-stranded overhangs, wherein each of the third plurality of partial double-stranded nucleic acid has a predetermined sequence different than another predetermined sequence in the third plurality of partial double-stranded nucleic acids.

In some embodiments, the method can further comprise assembling the library of variant nucleic acids by mixing the first, second and third pluralities of partial double-stranded nucleic acids under conditions sufficient to hybridize the complementary overhangs thereby forming the library of non-random variant target nucleic acids.

In some embodiments, the library generated can be a library of genes. In some embodiments, each double-stranded nucleic acid can have a size ranging from about 20 bases pairs to about 200 bases pairs.

In some embodiments, the library generated can be a library of genes. In some embodiments, each double stranded nucleic acid can have a size ranging from about 200 bases pairs to about 500 bases pairs.

Yet in other embodiments, the library generated can be a library of metabolic pathways. In some embodiments, each double-stranded nucleic acid can have a size ranging from about 500 bases pairs to about 3,000 bases pairs. In some embodiments, each double-stranded nucleic acid can be a gene or a set of genes. In some embodiments, each double-stranded nucleic acid can comprise a genetic element. In some embodiments, each double stranded nucleic acid can be an operon comprising a promoter sequence, a ribosomal binding site sequence, a gene or set of genes, a terminator or any combination thereof. In some embodiments, the library can be a library of operons comprising promoters having different strengths. In some embodiments, the library can be a library of operons comprising ribosomal binding sites having different strengths.

According to some disclosure herein, the method of generating a nucleic acid library comprises the steps of identifying a target nucleic acid, identifying in the target nucleic acid a first region, wherein the first region comprises a variant nucleic acid sequence; and identifying in the target nucleic acid a second region, wherein the second region comprises an invariant sequence. In some embodiments, the target nucleic acid can comprise one or more invariant or constant regions, one or more variable regions and a combination thereof.

The target nucleic acid can then be parsed in at least a first plurality of oligonucleotides comprising the variant nucleic acid sequence and at least a second plurality of oligonucleotides comprising the invariant nucleic acid sequence. The at least first and second pluralities of oligonucleotides can be provided and assembled. In some embodiments, the library can be assembled using a polymerase-based assembly reaction, ligase-based assembly reaction, or a combination thereof.

In some embodiments, the target nucleic acid can encode for a polypeptide having one or more domains. In some embodiments, the variant nucleic acid sequence can comprise a deletion of nucleic acid sequences encoding at least part of the one or more domains, an insertion of nucleic acid sequences encoding at least part of the one or more domains or a combination thereof. In some embodiments, the variant nucleic acid sequence can comprise any of the following: one or more deletion(s) of nucleic acid sequences, one or more insertion(s) of nucleic acid sequences, one or more substitution(s), or any combination of two or more of any of the foregoing. In some embodiments, the deletion(s) can be deletion(s) of nucleic acid sequences encoding at least part of one or more domains. In some embodiments, the insertion(s) can be insertion(s) of nucleic acid sequences encoding at least part of one or more domains. In some embodiments the substitution(s) can be substitution(s) of nucleotides in nucleic acid sequences encoding at least part of one or more domains. In some embodiments, the deletion(s), insertion(s), or substitutions (or any combination of any of the foregoing) can be one or more multiples of 3 nucleotides. In some embodiments, the deletion(s), insertion(s), or substitutions (or any combination of any of the foregoing) can comprise a single multiple of 3 consecutive nucleotides. In other embodiments, the deletion(s), insertion(s), or substitution(s) (or any combination of any of the foregoing) can comprise five or fewer multiples of 3 consecutive nucleotides. In some embodiments, the deletion(s), insertion(s), or substitutions (or any combination of any of the foregoing) can comprise 6 or fewer, 7 or fewer, 8 or fewer, 9 or fewer, 10 or fewer, 11 or fewer, 12 or fewer, or more multiples of 3 consecutive nucleotides. In some embodiments, substitution(s) can be a multiple of 3 consecutive nucleotides substitutions, or can encompass nucleotides in any number, including without limitation, one nucleotide, or two nucleotides, or more than two nucleotides.

In some embodiments, the target nucleic acid is a gene or set of genes. In some embodiments, the deletion(s), insertion(s), or substitution(s) (or any combination of the foregoing) is in the non-coding sequence of the gene or set of genes. In some embodiments, non-coding sequence of the gene or set of genes can comprise deletions(s), insertion(s), or substitution(s) (or any combination of any of the foregoing). Particularly when located in the non-coding sequence, deletion(s), insertion(s), or substitution(s) (or any combination of the foregoing) can comprise nucleotides in any number, including one or more multiples of 3 consecutive nucleotides. According to an embodiment of the disclosure, deletion(s), insertion(s), or substitution(s) (or any combination of any of the foregoing) may be found in a coding region, a non-coding region, or both.

In some embodiments, the method for producing a library of nucleic acids comprises selecting a target nucleic acid sequence, selecting at least a nucleic acid sequence to be deleted or inserted at one or more selected positions, designing a first set of oligonucleotides having variant sequences at the selected positions and at least a second set of oligonucleotides having an invariant sequence, and assembling the first and the at least second sets of oligonucleotides. In some embodiments, in the step of selecting, the nucleic acid sequence to be deleted, inserted, or substituted (or any combination of the foregoing) can be one or more multiples of 3 nucleotides. In some embodiments, in the step of selecting, the nucleic acid sequence to be deleted, inserted or substituted (or any combination of the foregoing) can comprise five or fewer multiples of 3 consecutive nucleotides. In some embodiments, in the step of selecting, the nucleic acid sequence to be deleted, inserted, or substituted (or any combination of the foregoing) can comprise 6 or fewer, 7 or fewer, 8 or fewer, 10 or fewer, 11 or fewer, 12 or fewer, or more multiples of 3 consecutive nucleotides. In some embodiments, substitution(s) can be a multiple of 3 consecutive nucleotides substitutions, or can encompass nucleotides in any number, including without limitation, one nucleotide, or two nucleotides, or more than two nucleotides.

In some embodiments, the first and second sets together can comprise the target nucleic acid sequence. In some embodiments, the first and second sets together can comprise a fragment of the target nucleic acid sequence. In some embodiments, the selected positions can comprise a nucleotide, a codon, a sequence of nucleotides or a combination thereof.

In some embodiments, the target nucleic acid is a gene or set of genes. In some embodiments, the deletion(s), insertion(s), or substitution(s) (or any combination of the foregoing) is in the non-coding sequence of the gene or set of genes. Particularly when located in the non-coding sequence, deletion(s), insertion(s), or substitutions (or any combination of the foregoing) can comprise nucleotides in any number, including one or more multiples of 3 nucleotides. According to an embodiment of the disclosure, insertions and/or deletions may be found in a coding region, a non-coding region, or both.

## BRIEF DESCRIPTION OF THE FIGURES

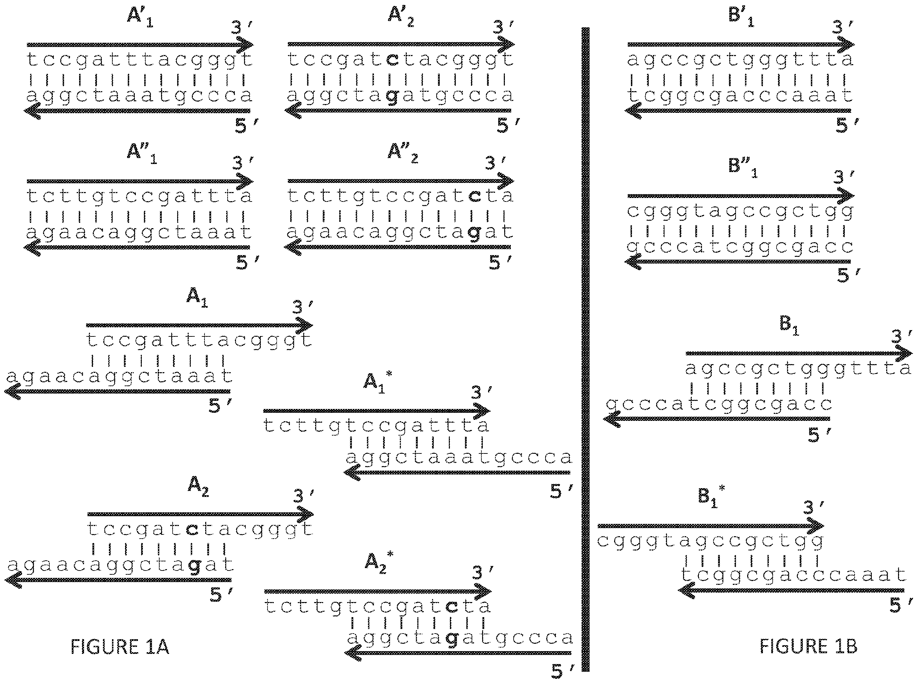

FIGS. 1A-1B illustrate a non-limiting exemplary method of the generation of overhang nucleic acids for use in building a non-random variant library. FIG. 1A shows the generation of nucleic acid duplexes with 3' overhangs in a first pool. FIG. 1B shows the generation of nucleic acid duplexes with 3' overhangs in a second pool.

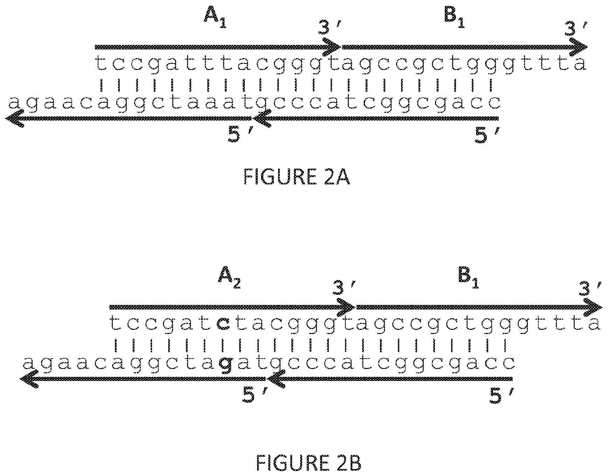

FIGS. 2A and 2B illustrate a non-limiting exemplary method of assembly of nucleic acid duplexes with overhangs for generating a non-random variant library.

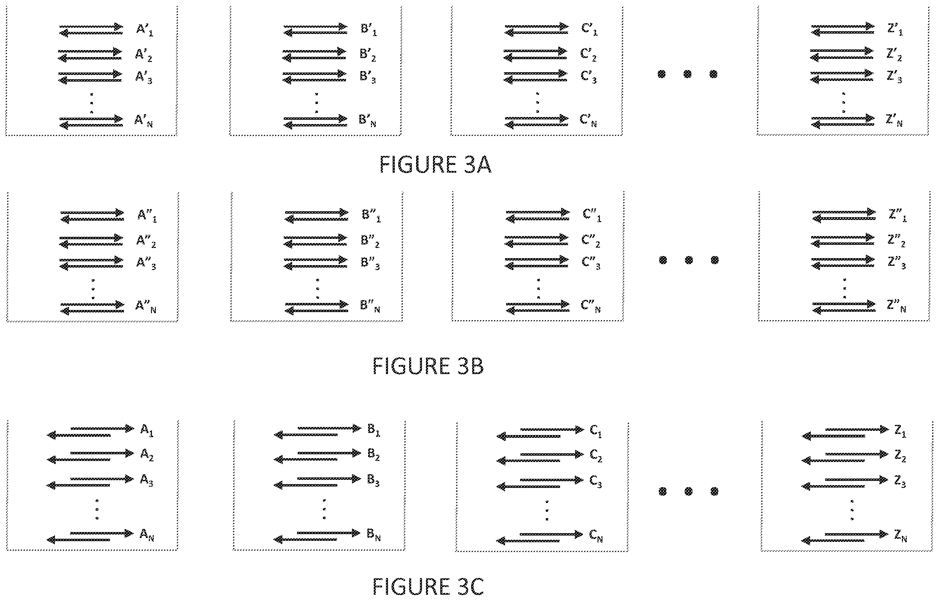

FIGS. 3A-3C illustrate a non-limiting exemplary method of building a non-random variant library. FIG.3A shows double-stranded library nucleic acids or fragments prepared in a first single reaction volume. FIG. 3B shows double-stranded library fragments prepared in a first single reaction volume. FIG. 3C shows the generation of a mixture of double stranded library fragments in a single volume.

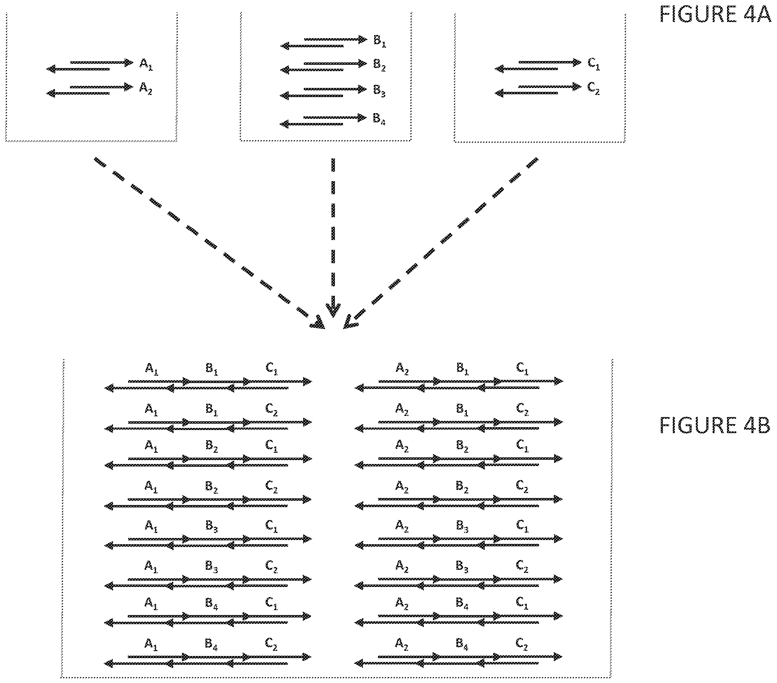

FIGS. 4A-B illustrate a non-limiting exemplary method of building a non-random variant library. FIG. 4A shows an embodiment in which two fragment A staggered hybridization products{A1, A2}, four fragment B staggered hybridization products{B1, B2, B3, B4}, and two fragment C staggered hybridization products{C1, C2} are combined to form a non-random library of nucleic acids. FIG. 4B shows the ligation of these sets of staggered hybridization products A, B, C in a single reaction volume.

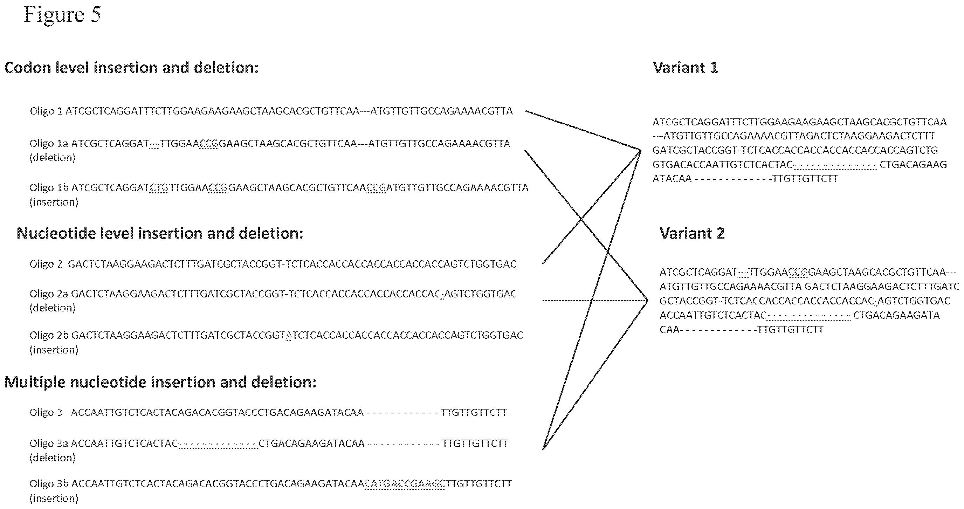

FIG. 5 illustrates a non-limiting embodiment of discrete synthesized sequences with deletion(s) and/or insertion(s) at the codon, nucleotide and multiple nucleotide levels and combinatorial assembly of such sequences. Deletions and insertions are underlined. Discrete sequences with deletion(s) and/or insertion(s) at the codon level were synthetized: oligo 1, oligo 1a with deletion of nucleotide CTG and insertion of 3 nucleotides CCG (underlined), oligo 1b with 3 nucleotides insertion CTG, 3 nucleotides insertion CCG (underlined) and 3 nucleotides insertion CCG (underlined). Discrete sequences with deletion(s) and/or insertion(s) at the nucleotide level were synthesized: oligo 2, oligo 2a with a single nucleotide deletion, oligo 2b with a single nucleotide A insertion (underlined). Discrete sequences with deletion(s) and/or insertion(s) at the multiple nucleotide level were synthetized: oligo 3, oligo 3a with 12 nucleotides deletion (underlined), oligo 3b with 12 nucleotides insertion (underlined). The oligonucleotides can be assembled into full variant constructs with the exact sequences as specified by the user: Variant 1: oligo 1 + oligo 2 + oligo 3a having the 12 nucleotides deletion and Variant 2: oligo 1a having the 3 nucleotides deletion and the 3 nucleotides insertion + oligo 2a having single nucleotide deletion + oligo 3a having the 12 nucleotides deletion.

## DETAILED DESCRIPTION OF THE INVENTION

Disclosures herein relate to methods and compositions for producing non-random nucleic acid libraries comprising a plurality of pre-selected or predetermined sequences of interest. Some disclosures herein relate to the chemical synthesis of libraries of nucleic acids for a wide range of applications including antibody design and metabolic pathway optimization. The general approach to making libraries of nucleic acids is to start with a single instance of the final product (e.g. a gene which might code for an antibody) and then to randomly mutate the gene such as by amplification with an error prone polymerase. Another approach to producing variant libraries is to introduce variation into DNA synthesis such as by coupling a mixture of nucleotide bases (e.g. a, c, t, and g) for particular coupling steps in a DNA synthesis reaction. A shortcoming of these approaches is that these methods produce random libraries which include a high number of library members which have a low likelihood of being variants of interest but which nonetheless need to be screened. In addition, such methods can take up a substantial fraction of the available screening resource.

Disclosures herein relate to methods for rationally designing and producing rationally designed variant libraries in which substantially every member or a substantial proportion of the members of the library is designed or engineered to have a non-random sequence. Such methods can limit the number of library members that are synthesized and screened, making good use of the available library screening resource. Accordingly, disclosures herein relate to methods and compositions that can reduce the complexity of libraries of variant nucleic acids, therefore reducing oversampling of these libraries during screening and improving screening efficiency.

Disclosures herein can be incorporated into nucleic assembly procedures to, for example, increase assembly fidelity, throughput and/or efficiency, decrease cost, and/or reduce assembly time. In some embodiments, disclosures herein may be automated and/or implemented in a high throughput assembly context to facilitate parallel production of many different variants of a target nucleic acid sequence.

As used herein the terms "nucleic acid", "polynucleotide", and "oligonucleotide" are used interchangeably and refer to naturally-occurring or synthetic polymeric forms of nucleotides. The oligonucleotides and nucleic acid molecules of the present disclosure may be formed from naturally occurring nucleotides, for example forming deoxyribonucleic acid (DNA) or ribonucleic acid (RNA) molecules. In some embodiments, the oligonucleotides and nucleic acid molecules may be methylated. Alternatively, the naturally occurring oligonucleotides may include structural modifications to alter their properties, such as in peptide nucleic acids (PNA) or in locked nucleic acids (LNA). The solid phase synthesis of oligonucleotides and nucleic acid molecules with naturally occurring or artificial bases is well known in the art. The terms should be understood to include equivalents, analogs of either RNA or DNA made from nucleotide analogs and as applicable to the embodiment being described, single-stranded or double-stranded polynucleotides. Nucleotides useful in the disclosure include, for example, naturally-occurring nucleotides (for example, ribonucleotides or deoxyribonucleotides), or natural or synthetic modifications of nucleotides, or artificial bases. As used herein, the term monomer refers to a member of a set of small molecules which are and can be joined together to form an oligomer, a polymer or a compound composed of two or more members. The particular ordering of monomers within a polymer is referred to herein as the "sequence" of the polymer. The set of monomers includes, but is not limited to, for example, the set of common L-amino acids, the set of D-amino acids, the set of synthetic and/or natural amino acids, the set of nucleotides and the set of pentoses and hexoses. Disclosures herein are described herein primarily with regard to the preparation and use of oligonucleotides, but could readily be applied in the preparation of other polymers such as peptides or polypeptides, polysaccharides, phospholipids, heteropolymers, polyesters, polycarbonates, polyureas, polyamides, polyethyleneimines, polyarylene sulfides, polysiloxanes, polyimides, polyacetates, or any other polymers.

The term "gene" refers to a nucleic acid fragment that expresses a specific protein, including regulatory sequences, for example regulatory sequences preceding (5' noncoding sequences) and following (3' non-coding sequences) the coding sequence.

"Promoter" refers to a nucleotide sequence capable of controlling the expression of a coding sequence or functional RNA. In general, a coding sequence is located 3' to a promoter sequence.

As used herein, the term "predetermined sequence", "predefined sequence" or "pre-selected sequence" are used interchangeably and means that the sequence of the polymer is known and chosen before synthesis or assembly of the polymer. In particular, disclosures herein are described herein primarily with regard to the preparation of nucleic acid molecules, the sequence of the nucleic acids being known and chosen before the synthesis or assembly of the nucleic acid molecules. In some embodiments of the technology provided herein, immobilized oligonucleotides or polynucleotides are used as a source of material. In various embodiments, the methods described herein use synthetic oligonucleotides, their sequence being determined based on the sequence of the final polynucleotide constructs to be synthesized. In one embodiment, oligonucleotides are short nucleic acid molecules. For example, oligonucleotides may be from 10 to about 300 nucleotides, from 20 to about 400 nucleotides, from 30 to about 500 nucleotides, from 40 to about 600 nucleotides, or more than about 600 nucleotides long. However, shorter or longer oligonucleotides may be used. Oligonucleotides may be designed to have different length. In some embodiments, the sequence of the polynucleotide construct may be divided up into a plurality of shorter sequences that can be synthesized in parallel and assembled into a single or a plurality of desired polynucleotide constructs using the methods described herein. In some embodiments, the assembly procedure may include several parallel and/or sequential reaction steps in which a plurality of different nucleic acids or oligonucleotides are synthesized or immobilized, primer-extended or amplified, and are combined in order to be assembled (e.g., by extension or ligation as described herein) to generate a longer nucleic acid product to be used for further assembly, cloning, or other applications.

A "non-random" library of nucleic acid sequences as used herein means that the target nucleic acid sequences in the library are substantially pre-selected or predetermined prior to assembly, as opposed as being degenerated or randomly derived. As used herein the term "non-random variant libraries" and "Variant Libraries by Multiplexed Polynucleotide Synthesis (VL-MPS)"are used interchangeably. In some embodiments, non-random libraries according to disclosures herein are substantially free of random sequence variations (e.g. contains less than 10%, less than 5%, less than 1%, less than 0.1%, or less than 0.01% of random variations). One of skill in the art will appreciate that variant nucleic acids can include any of a variety of sites of variation of a reference nucleic acid sequence to be varied.

In some embodiments, variant members of the non-random library may be related sequences that comprise single or multiple sequence variations based on a predetermined reference sequence. According to some disclosure herein, a non-random library may be assembled from a plurality of nucleic acids (e.g., polynucleotides, oligonucleotides, etc.) to form a longer nucleic acid product. A library may contain nucleic acids that include identical (non-variant) regions and regions of sequence variation. Accordingly, certain nucleic acids being assembled may correspond to the non-variant sequence regions while other nucleic acids being assembled may correspond to one of several predetermined sequence variants in a predetermined region of sequence variation. In some embodiments, the non-random nucleic acid libraries can comprise two or more nucleic acids that encode two or more polypeptides of interest. In some embodiments, the non-random library may be designed to express any type of polypeptide, for example scaffold proteins, antibodies, enzymes etc....

Synthetic oligonucleotides

In some embodiments, the methods and devices provided herein use oligonucleotides that are immobilized on a surface or substrate (e.g., support-bound oligonucleotides). Support-bound oligonucleotides comprise for example, oligonucleotides complementary to construction oligonucleotides, anchor oligonucleotides and/or spacer oligonucleotides. As used herein the terms "support", "substrate" and "surface" are used interchangeably and refer to a porous or non-porous solvent insoluble material on which polymers such as nucleic acids are synthesized or immobilized. As used herein "porous" means that the material contains pores having substantially uniform diameters (for example in the nm range). Porous materials include paper, synthetic filters etc. In such porous materials, the reaction may take place within the pores. The support can have any one of a number of shapes, such as pin, strip, plate, disk, rod, bends, cylindrical structure, particle, including bead, nanoparticles and the like. The support can have variable widths. The support can be hydrophilic or capable of being rendered hydrophilic and includes inorganic powders such as silica, and materials derived from cellulose, such as fiber containing papers, e.g., filter paper, chromatographic paper, etc.; synthetic or modified naturally occurring polymers, such as nitrocellulose, cellulose acetate, poly (vinyl chloride), polyacrylamide, cross linked dextran, agarose, polyacrylate, polyethylene, polypropylene, poly (4-methylbutene), polystyrene, polymethacrylate, poly(ethylene terephthalate), nylon, poly(vinyl butyrate), polyvinylidene difluoride (PVDF) membrane, glass, controlled pore glass, magnetic controlled pore glass, ceramics, metals, and the like etc.; either used by themselves or in conjunction with other materials. In some embodiments, oligonucleotides are synthesized in an array format. For example, single-stranded oligonucleotides are synthesized in situ on a common support, wherein each oligonucleotide is synthesized on a separate or discrete feature (or spot) on the substrate. In an embodiment, single-stranded oligonucleotides are bound to the surface of the support or feature. As used herein the term "array" refers to an arrangement of discrete features for storing, amplifying and releasing oligonucleotides or complementary oligonucleotides for further reactions. In a preferred embodiment, the support or array is addressable: the support includes two or more discrete addressable features at a particular predetermined location (i.e., an "address") on the support. Therefore, each oligonucleotide molecule on the array is localized to a known and defined location on the support. The sequence of each oligonucleotide can be determined from its position on the support. The array may comprise interfeatures regions. Interfeatures may not carry any oligonucleotide on their surface and may correspond to inert space.

In some embodiments, oligonucleotides are attached, spotted, immobilized, surface-bound, supported or synthesized on the discrete features of the surface or array.

Some disclosures herein relate to a polynucleotide assembly process wherein synthetic oligonucleotides are designed and used as templates for primer extension reactions, synthesis of complementary oligonucleotides and to assemble polynucleotides into longer polynucleotides constructs. In some embodiments, the method includes synthesizing a plurality of oligonucleotides or polynucleotides in a chain extension reaction using a first plurality of single-stranded oligonucleotides as templates. As noted above, the oligonucleotides may be first synthesized onto a plurality of discrete features of the surface, or on a plurality of supports (e.g., beads) or may be deposited on the plurality of features of the support or on the plurality of supports. The support may comprise at least 100, at least 1,000, at least $10^4$, at least $10^5$, at least $10^6$, at least $10^7$, or at least $10^8$ features. In some embodiments, the oligonucleotides are covalently attached to the support. In some embodiments, the pluralities of oligonucleotides are immobilized to a solid surface.

In some embodiments, the support-bound oligonucleotides may be attached through their 5' end. Yet in other embodiments, the support-bound oligonucleotides are attached through their 3' end. In some embodiments, the support-bound oligonucleotides may be immobilized on the support via a nucleotide sequence (e.g., degenerate binding sequence), linker or spacer (e.g., photocleavable linker or chemical linker). It should be appreciated that by 3' end, it is meant the sequence downstream to the 5' end and by 5' end it is meant the sequence upstream to the 3' end. For example, an oligonucleotide may be immobilized on the support via a nucleotide sequence, linker or spacer that is not involved in hybridization. The 3' end sequence of the support-bound oligonucleotide refers then to a sequence upstream to the linker or spacer.

In certain embodiments, oligonucleotides may be designed to have a sequence that is identical or complementary to a different portion of the sequence of a predetermined target polynucleotide that is to be assembled. Accordingly, in some embodiments, each oligonucleotide may have a sequence that is identical or complementary to a portion of one of the two strands of a double-stranded target nucleic acid. As used herein, the term "complementary" refers to the capacity for precise pairing between two nucleotides. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. The term "orthogonal" means that the sequences are different, non-interfering, or non-complementary.

In some embodiments, a plurality of conduction oligonucleotides is provided. In some embodiments, the construction oligonucleotides are synthesized using support-bound oligonucleotides as templates.

In some embodiments, the plurality of construction oligonucleotides are designed such that each plurality of construction oligonucleotides comprises a sequence region at its 5' end that is complementary to sequence region of the 5' end of another construction oligonucleotide and a sequence region at its 3' end that is complementary to a sequence region at a 3' end of a different construction oligonucleotide. In some embodiments, the plurality of construction oligonucleotides are designed such that each plurality of construction oligonucleotides comprises a sequence region at its 5' end that is identical to a sequence region of the 5' end of another construction oligonucleotide and a sequence region at its 3' end that is identical to a sequence region at a 3' end of a different construction oligonucleotide. As used herein, a "construction" oligonucleotide refers to one of the plurality or population of single-stranded or double-stranded oligonucleotides used for the generation of offset dimers for nucleic acid assembly. The plurality of construction oligonucleotides can be double-stranded and can comprise oligonucleotides for both the sense and antisense strand of the target polynucleotide. Construction oligonucleotides can be blunt-end oligonucleotide duplexes. Construction oligonucleotides can have any length, the length being designed to accommodate an overlap or complementary sequence. Construction oligonucleotides can be of identical size or of different sizes. In preferred embodiments, the construction oligonucleotides span the entire sequence of the target polynucleotide without any gaps. Yet in other embodiments, the construction oligonucleotides are partially overlapping, resulting in gaps between construction oligonucleotides when hybridized to each other. In some embodiments, the construction oligonucleotides can have additional sequences than the target polynucleotide sequence. For example, the construction oligonucleotides can be modified construction oligonucleotides having an insertion and/or a deletion. In some embodiments, the construction oligonucleotides can have one or more substitutions. In some embodiments, the construction oligonucleotides can have one or more insertion(s), one or more deletion(s), one or more substitution(s), or any combination of the foregoing. In some embodiments, the pool or population of construction oligonucleotides comprises construction oligonucleotides having overlapping sequences (complementary or identical).

As used herein, the term "dimer" refers to an oligonucleotide duplex or double-stranded oligonucleotide molecule. The term "offset dimer" and "offset duplex" are used interchangeably and refer to an oligonucleotide duplex having a 3' and/or 5' overhang (or cohesive ends, i.e., non-blunt end). In some embodiments, the offset dimers are partially double-stranded nucleic acids (e.g. oligonucleotides) whereby the nucleic acids comprise a first single-stranded overhang and a second single-stranded overhang. For example, the offset dimer can have a 3' overhang or the offset dimer can have a 5' overhang.

In some embodiments, the offset dimers are generated by denaturation and re-hybridization of construction oligonucleotides in a pool.

It should be appreciated that different oligonucleotides may be designed to have different lengths with overlapping sequence regions. Overlapping sequence regions may be identical (i.e., corresponding to the same strand of the nucleic acid fragment) or complementary (i.e., corresponding to complementary strands of the nucleic acid fragment). Overlapping sequences may be of any suitable length. Overlapping sequences may be between about 5 and about 500 nucleotides long (e.g., between about 10 and 100, between about 10 and 75, between about 10 and 50, about 20, about 25, about 30, about 35, about 40, about 45, about 50, etc... nucleotides long) However, shorter, longer or intermediate overlapping lengths may be used. It should be appreciated that overlaps (5' or 3' regions) between different input nucleic acids used in an assembly reaction may have different lengths.

In some embodiments, nucleic acids are assembled using ligase-based assembly techniques. In some embodiments, oligonucleotides are designed to provide full length sense (or plus strand) and antisense (or minus strand) strands of the target polynucleotide construct. After hybridization of sense and antisense oligonucleotides to form offset dimers, the offset dimers are subjected to ligation in order to form the target polynucleotide construct or a sub-assembly product. Reference is made to U.S. Pat. No. 5,942,609. Ligase-based assembly techniques may involve one or more suitable ligase enzymes that can catalyze the covalent linking of adjacent 3' and 5' nucleic acid termini (e.g., a 5' phosphate and a 3' hydroxyl of nucleic acid(s) annealed on a complementary template nucleic acid such that the 3' terminus is immediately adjacent to the 5' terminus). Accordingly, a ligase may catalyze a ligation reaction between the 5' phosphate of a first nucleic acid to the 3' hydroxyl of a second nucleic acid if the first and second nucleic acids are annealed next to each other on a template nucleic acid. A ligase may be obtained from recombinant or natural sources. A ligase may be a heat-stable ligase. In some embodiments, a thermostable ligase from a thermophilic organism may be used. Examples of thermostable DNA ligases include, but are not limited to: Tth DNA ligase (from Thermus thermophilus, available from, for example, Eurogentec and GeneCraft); Pfu DNA ligase (a hyperthermophilic ligase from Pyrococcus furiosus); Taq ligase (from Thermus aquaticus), Ampliligase® (available from Epicenter Biotechnologies) any other suitable heat-stable ligase, or any combination thereof. In some embodiments, one or more lower temperature ligases may be used (e.g., T4 DNA ligase). A lower temperature ligase may be useful for shorter overhangs (e.g., about 3, about 4, about 5, or about 6 base overhangs) that may not be stable at higher temperatures. Non-enzymatic techniques, for example chemical ligation, can be used to ligate nucleic acids.

## Multiplex Polynucleotide Synthesis

Disclosures herein relate to the chemical synthesis of libraries of nucleic acids for a wide range of applications. Some embodiments of the disclosure relate to quick and inexpensive methods for the synthesis of nucleic acid libraries. It should be appreciated that a significant part of the cost of polynucleotide synthesis is the cost of the reagents for carrying out the polynucleotide synthesis reactions. In order to lower this cost, reactions may be carried out in smaller volumes. In some embodiments, reactions may be carried out in individual microvolumes, such as droplets. According to some disclosure herein, a plurality of different nucleic acids can be synthesized within a single synthesis reaction volume in a multiplexed nucleic acid synthesis. One of skill in the art will appreciate that the library may be assembled by serial, parallel or hierarchical multiplexed assembly process. In some embodiments, the library may be assembled in a single reaction or intermediate nucleic acid fragments may be assembled separately and then combined in one or more round of assembly (e.g. hybridization and ligation).

It should be appreciated that, in a first step, construction nucleic acid sequences or construction oligonucleotides are designed. Construction nucleic acids may be synthetic oligonucleotides, as described herein, amplification products, restriction fragments or other suitable nucleic acids. In some embodiments, certain construction nucleic acids may include one or more sequence variations. In some embodiments, the construction nucleic acids may be designed such that the 5' end of a first construction nucleic acid in a first pool is identical to the 3' end of a second construction nucleic acid in a second pool.

According to some disclosure herein, a non-random library may be assembled by combining two or more pools of nucleic acids, each nucleic acid having a predetermined sequence. In some embodiments, one or more pools may have nucleic acid variant sequences. For example, the nucleic acid library may be assembled by combining one pool of nucleic acid variants with one pool of nucleic acids having non-variable (or constant) sequences. Yet in other embodiments, the nucleic acid library may be assembled by combining a plurality of pools of nucleic acid variants. Accordingly, different libraries with different types of variants or different density of variants may be designed and assembled.

In some embodiments, the concentration of each nucleic acid that is combined can be adjusted to improve the assembly reaction and drive the reactions to the formation of the full length nucleic acids. In some embodiments, the concentration of each nucleic acid is biased so as to change the ratio of the represented nucleic acid variants. In some embodiments, each construction nucleic acid can be added in a pre-defined ratio so as to bias the resulting nucleic acid library. For example, if it is desired that the library has a certain level of a specific variation(s) and a lesser level of another variation(s) at the same or different site, the library may be biased by adding greater levels of the desired variation(s).

In some embodiments, nucleic acids having variable sequences can be combined with the nucleic acids having non-variable sequences in a predefined ratio so as to bias the nucleic acid library.

Certain embodiments of multiplex nucleic acid assembly reactions for generating libraries of nucleic acids having a predetermined sequence are illustrated with reference to FIGS. 1-4. It should be appreciated that synthesis and assembly methods described herein (including, for example, oligonucleotide synthesis, step-wise assembly, multiplex nucleic acid assembly, hierarchical assembly of nucleic acid fragments, or any combination thereof) may be performed in any suitable format, including in a reaction tube, in a multi-well plate, on a surface, on a column, in a microfluidic device (e.g., a microfluidic tube), a capillary tube, etc.

A predetermined nucleic acid member of the library may be assembled from a plurality of different starting nucleic acids (e.g., oligonucleotides) in a multiplex assembly reaction (e.g., a multiplex enzyme-mediated reaction, a multiplex chemical assembly reaction, or a combination thereof). Certain aspects of multiplex nucleic acid assembly reactions are illustrated by the following description of certain embodiments of multiplex oligonucleotide assembly reactions. It should be appreciated that the description of the assembly reactions in the context of oligonucleotides is not intended to be limiting. The assembly reactions described herein may be performed using starting nucleic acids obtained from one or more different sources (e.g., synthetic or natural polynucleotides, nucleic acid amplification products, nucleic acid degradation products, synthetic or natural oligonucleotides, synthetic or natural genes, etc.). The starting nucleic acids may be referred to as assembly nucleic acids (e.g., assembly oligonucleotides). As used herein, an assembly nucleic acid or an offset dimer has a sequence that is designed to be incorporated into the nucleic acid product generated during the assembly process. However, it should be appreciated that the description of the assembly reactions in the context of double-stranded nucleic acids is not intended to be limiting. In some embodiments, one or more of the starting nucleic acids illustrated in the figures and described herein may be provided as single-stranded nucleic acids. Accordingly, it should be appreciated that where the figures and description illustrate the assembly of cohesive-end double-stranded nucleic acids, the presence of one or more single-stranded nucleic acids is contemplated.

According to various embodiments, the target nucleic acids can be divided first into two or more overlapping nucleic acid fragments (or subassembly fragments). Each nucleic acid fragment is then subdivided into two or more overlapping smaller nucleic acid fragments.

Oligonucleotides may be synthesized using any suitable technique. For example, oligonucleotides may be synthesized on a column or other support (e.g., a chip or array). Examples of chip-based synthesis techniques include techniques used in synthesis devices or methods available from CombiMatrix, Agilent, Affymetrix, or other sources. A synthetic oligonucleotide may be of any suitable size, for example between 10 and 1,000 nucleotides long (e.g., between 10 and 200, 200 and 500, 500 and 1,000 nucleotides long, or any combination thereof). An assembly reaction may include a plurality of oligonucleotides, each of which independently may be between 10 and 300 nucleotides in length (e.g., between 20 and 250, between 30 and 200, 50 to 150, 50 to 100, or any intermediate number of nucleotides). However, one or more shorter or longer oligonucleotides may be used in certain embodiments.

As used herein, an oligonucleotide may be a nucleic acid molecule comprising at least two covalently bonded nucleotide residues. In some embodiments, an oligonucleotide may be between 10 and 1,000 nucleotides long. For example, an oligonucleotide may be between about 10 and about 500 nucleotides long, or between about 500 and about 1,000 nucleotides long. In some embodiments, an oligonucleotide may be between about 20 and about 300 nucleotides long (e.g., from about 30 to 250, 40 to 220, 50 to 200, 60 to 180, or about 65 or about 150 nucleotides long), between about 100 and about 200, between about 200 and about 300 nucleotides, between about 300 and about 400, or between about 400 and about 500 nucleotides long. However, shorter or longer oligonucleotides may be used. An oligonucleotide may be a single-stranded nucleic acid. However, in some embodiments a double-stranded oligonucleotide may be used as described herein. In certain embodiments, an oligonucleotide may be chemically synthesized as described in more detail below. In some embodiments, an input nucleic acid (e.g., synthetic oligonucleotide or nucleic acid fragment) may be amplified before use. The resulting product may be double-stranded.

In certain embodiments, each oligonucleotide may be designed to have a sequence that is identical to a different portion of the sequence of a predetermined target nucleic acid that is to be assembled. Accordingly, in some embodiments each oligonucleotide may have a sequence that is identical to a portion of one of the two strands of a double-stranded target nucleic acid. For clarity, the two complementary strands of a double stranded nucleic acid are referred to herein as the positive (P) and negative (N) strands. This designation is not intended to imply that the strands are sense and anti-sense strands of a coding sequence. They refer only to the two complementary strands of a nucleic acid (e.g., a target nucleic acid, an intermediate nucleic acid fragment, etc.) regardless of the sequence or function of the nucleic acid. Accordingly, in some embodiments a P strand may be a sense strand of a coding sequence, whereas in other embodiments a P strand may be an anti-sense strand of a coding sequence. It should be appreciated that the reference to complementary nucleic acids or complementary nucleic acid regions herein refers to nucleic acids or regions thereof that have sequences which are reverse complements of each other so that they can hybridize in an antiparallel fashion typical of natural DNA.

According to disclosure herein, a target nucleic acid may be the P strand, the N strand, or a double-stranded nucleic acid comprising both the P and N strands. It should be appreciated that different oligonucleotides may be designed to have different lengths. In some embodiments, one or more different offset oligonucleotides may have overlapping sequence regions or overhangs (e.g., overlapping 5' regions and/or overlapping 3' regions). Overlapping sequence regions may be identical (i.e., corresponding to the same strand of the nucleic acid fragment) or complementary (i.e., corresponding to complementary strands of the nucleic acid fragment). The plurality of offset oligonucleotide dimers may include one or more oligonucleotide pairs with identical overlapping sequence regions, one or more oligonucleotide pairs with overlapping complementary sequence regions, or a combination thereof. Overlapping sequences may be of any suitable length. For example, overlapping sequences may encompass the entire length of one or more nucleic acids used in an assembly reaction. Overlapping sequences may be between about 2 and about 50 (e.g., between 3 and 20, between 3 and 10, between 3 and 8, or 4, 5, 6, 7, 8, 9, etc. nucleotides long). However, shorter, longer or intermediate overlapping lengths may be used. It should be appreciated that overlaps between different offset oligonucleotide dimers used in an assembly reaction may have different lengths and/or sequences. For example, the overlapping sequences may be different from one another by at least one nucleotide, 2 nucleotides, 3 nucleotides, or more.

In a multiplex oligonucleotide assembly reaction designed to generate a predetermined nucleic acid fragment, the combined sequences of the different oligonucleotides in the reaction may span the sequence of the entire nucleic acid fragment on either the positive strand, the negative strand, both strands, or a combination of portions of the positive strand and portions of the negative strand. The plurality of different oligonucleotides may provide either positive sequences, negative sequences, or a combination of both positive and negative sequences corresponding to the entire sequence of the nucleic acid fragment to be assembled.

In disclosure herein, a nucleic acid fragment may be assembled in a ligase-mediated assembly reaction from a plurality of oligonucleotides that are combined and ligated in one or more rounds of ligase-mediated ligations. Ligase-based assembly techniques may involve one or more suitable ligase enzymes that can catalyze the covalent linking of adjacent 3' and 5' nucleic acid termini (e.g., a 5' phosphate and a 3' hydroxyl of nucleic acid(s) annealed on a complementary template nucleic acid such that the 3' terminus is immediately adjacent to the 5' terminus). Accordingly, a ligase may catalyze a ligation reaction between the 5' phosphate of a first nucleic acid and the 3' hydroxyl of a second nucleic acid if the first and second nucleic acids are annealed next to each other on a template nucleic acid.

One should appreciate that the multiplex polynucleotide assembly reactions can take place in a single volume, for example in a well, or can take place in a localized individual microvolume. In some embodiments, the extension and/or assembly reactions are performed within a microdroplet (see PCT Application PCT/US2009/55267 and PCT Application PCT/US2010/055298).

Library construction

Some disclosures herein relate to the design and production of offset duplex (also referred herein as offset dimers) having cohesive ends and for assembly of the offset duplexes to form variants libraries. FIGS. 1A-1B show an exemplary method for Multiplexed Offset Duplex (or Dimers) Preparation. FIGS. 1A-1B illustrate the multiplexed preparation of the offset dimer building blocks (also referred herein as double-stranded overhanging oligonucleotides).

In some embodiments, a first and at least a second plurality of double-stranded overhanging nucleic acids are generated as building blocks for the assembly of a non-random library of nucleic acids. In some embodiments, each nucleic acid from the library is assembled by hybridization and ligation of nucleic acids having complementary overhangs (or cohesive ends).

According to some disclosure herein, the method comprises providing a first population of partially double-stranded oligonucleotides, whereby each first oligonucleotide comprises a first and a second single-stranded overhang, and providing a second population of partially double-stranded oligonucleotides, whereby each second oligonucleotide comprises a first single-stranded overhang and a second single-stranded overhang. In some embodiments, the first overhangs in the first population are identical, and the second overhangs in the first population are identical. In some embodiments, the identical first overhang of the first population of oligonucleotides is complementary to the identical first overhang of the population of second oligonucleotides. According to some disclosure herein, the first oligonucleotides can be ligated to the second oligonucleotides via the single-stranded overhang of the first oligonucleotide and the single-stranded overhang of the second oligonucleotide, generating a first ligation product. The first ligation product can contain the first overhang of the first oligonucleotide and the second overhang of the second oligonucleotide.

Referring to FIG 1A, a first plurality of nucleic acids (A) with staggered overhangs are generated. In some embodiments, the construction oligonucleotides can be amplified from template support-bound oligonucleotides. For example, oligonucleotides

$$[A']_1,$$

$$⟦A'⟧_2, ⟦A''⟧_1, ⟦A''⟧_2$$

can be amplified from template oligonucleotides to form a plurality of blunt end double-stranded oligonucleotides in a single first reaction volume. One should appreciate that the plurality of double-stranded construction oligonucleotides may be obtained from a commercial source or may be designed and/or synthesized onto a solid support (e.g. array). However, it should be appreciated that other nucleic acids (e.g., single or double-stranded nucleic acid degradation products, restriction fragments, amplification products, naturally occurring small nucleic acids, other polynucleotides, etc.) can be used.

In some embodiments, the oligonucleotides of a first set of blunt-end double-stranded oligonucleotides (e.g.

$$⟦A'⟧_1, ⟦A''⟧_1$$

are designed so that each sequence is offset from another sequence of the set by n bases. In some embodiments, the offset n may range from 2 to 8 bases. For example, the offset can be 2 bases, 3 bases, 4 bases, 5 bases, 6 base, 7 base, 8 bases or more. For example, referring to FIG. 1A, the oligonucleotides are designed so that the first set of blunt-end double-stranded oligonucleotides

$$⟦A'⟧_1$$

and

$$⟦A''⟧_1$$

as well as the second set of blunt-end double-stranded oligonucleotides

$$⟦A'⟧_2$$

and

$$⟦A''⟧_2$$

have sequences which are offset from each other by 4 bases.

In some embodiments, a second set of blunt-end double-stranded oligonucleotides is provided. In some embodiments, the blunt-end double-stranded oligonucleotides of the second set of blunt-end double-stranded oligonucleotides can be a sequence variant of the blunt-end double-stranded oligonucleotides of the first set of blunt-end double-stranded oligonucleotides. For example, the second set of oligonucleotides can contain a mutation, substitution, etc... The mutations can be at predetermined sites or at random sites. In some embodiments, the second set of blunt-end double-stranded oligonucleotides comprises nucleic acids from a nucleic acid variant library. In some embodiments, the nucleic acid variant library can be designed from a reference gene and can contain a predetermined number of mutations (n). The mutations within each set can be at the same or different position; and at any position.

In some embodiments, the blunt end double-stranded oligonucleotides in each set can be subjected to conditions promoting denaturation (e.g. by raising the temperature to a temperature above the melting temperature) and are then allowed to re-hybridize to form double-stranded oligonucleotides having overhangs.

Referring to the bottom of FIG. 1A, the double stranded oligonucleotides

$$⟦A'⟧_1$$

(SEQ ID NO: 1),

$$⟦A'⟧_2$$

(SEQ ID NO: 2),

$$⟦A''⟧_1$$

(SEQ ID NO; 3),

$$⟦A''⟧_2$$

(SEQ ID NO; 4) can be de-hybridized or denatured (e.g. by melting) and re-hybridized to form staggered hybridization products. The double-stranded oligonucleotides with overhangs can have, according to some embodiments, different internal double-stranded sequence but identical single-stranded overhangs. Still referring to FIG. 1A, the offset dimer products (e.g. $A_1$ and $A_2$) can have identical n base overhangs (e.g. 3' end overhangs) but may have different internal sequences. As shown in FIG. 1A, the offset dimerAi has a sequence (tccgatttacgggt, SEQ ID NO: 1) that differs from the offset dimer $A_2$ (tccgatctacgggt, SEQ ID NO: 2) in the presence of a 't' nucleotide instead of a 'c' nucleotide. Referring to FIG. 1A, the hybridization produces products A1 (SEQ ID NO: 1, SEQ ID NO: 7) and A2 (SEQ ID NO: 2 and SEQ ID NO: 8). The hybridization reaction can also produce products $A_1$* (SEQ ID NO: 1, SEQ ID NO: 9) and $A_2$* (SEQ ID NO: 2, SEQ ID NO: 10).

Referring to FiG. 1B, a second plurality of nucleic acids (B) with staggered overhangs can be generated following the same methods described for the first plurality of nucleic acids (e.g. nucleic acids A). Upon denaturation and re-hybridization, the nucleic acids can form partially double-stranded nucleic acids having single-stranded overhangs. For example, as illustrated in Figure 1B, nucleic acid $B_1$ (SEQ ID NO: 5, SEQ ID NO: 11) having a 3' overhang can be formed. In addition, nucleic acids $B_1$* (SEQ ID NO: 6, SEQ ID NO: 12) having a 5' overhang can also be formed.

FIGS. 2A-2B illustrate a non-limiting example of the assembly of two nucleic acid variants using three offset dimers. According to some embodiments, the nucleic acids having complementary overhangs can hybridize to form gapless ligatable junctions and can be ligated to form a longer nucleic acid sequence. For example, nucleic acids having a 3' overhang can hybridize with nucleic acids having a complementary 3' single-stranded overhang. Referring to FIGS. 2A-2B, a variant library can be generated by mixing and assembling the nucleic acids with complementary overhangs of FIG. 1. Still referring to FIGS. 2A-2B, offset dimer B1 having overhangs complementary to variants $A_1$ and $A_2$ can be ligated to variants $A_1$ (FIG. 2A) and $A_2$ (FIG. 2B) in a single reaction volume, to form variant library products $A_1 B_1$ (SEQ ID NO: 13, SEQ ID NO: 14) and $A_2 Bi$ (SEQ ID NO: 15, SEQ ID NO: 16).

Disclosures herein relate to the synthesis of complex variant libraries. FIGS. 3A-3C and FIGS.4A-4B illustrate embodiments to produce a more complex variant library by multiplex polynucleotide assembly. Referring to FIG.3A, double-stranded library nucleic acids or fragments

$$\{⟦A'⟧_1, ⟦A'⟧_2, ⟦A'⟧_3 \ldots ⟦A'⟧_N\}$$

can be prepared in a first single reaction volume. For example, the double-stranded nucleic acids can be synthesized by amplification of support bound oligonucleotides on an array. Double-stranded library fragments

$$\{⟦B'⟧_1, ⟦B'⟧_2, ⟦B'⟧_3 \ldots$$

$$⟦B'⟧_N\}$$

can be prepared in a second single reaction volume, and double-stranded library fragments

$$\{⟦C'⟧_1, ⟦C'⟧_2, ⟦C'⟧_3 \ldots ⟦C'⟧_N\}$$

can be prepared in a third reaction volume etc.

Referring to FIG. 3B, double-stranded library fragments

$$\{ \llbracket A'' \rrbracket_1, \llbracket A'' \rrbracket_2, \llbracket A'' \rrbracket_3 \ldots$$

$$\llbracket A'' \rrbracket_N \}$$

can be prepared in a first single reaction volume. In an exemplary embodiment, double-stranded oligonucleotides can be amplified using template support bound oligonucleotides on an array. Double-stranded library fragments

$$\{ \llbracket B'' \rrbracket_1, \llbracket B'' \rrbracket_2, \ldots \llbracket B'' \rrbracket_N \}$$

can be prepared in a second single reaction volume,

$$\{ \llbracket C'' \rrbracket_1, \llbracket C'' \rrbracket_2, \llbracket C'' \rrbracket_3 \ldots \llbracket C'' \rrbracket_N \}$$

can be prepared in a third reaction volume etc.

Referring to FIG. 3C double stranded library fragments

$$\{ \llbracket A' \rrbracket_1, \llbracket A' \rrbracket_2, \llbracket A' \rrbracket_3 \ldots$$

$$\llbracket A' \rrbracket_N \}$$

are combined with double stranded library fragments

$$\{ \llbracket A'' \rrbracket_1, \llbracket A'' \rrbracket_2, \llbracket A'' \rrbracket_3 \ldots \llbracket A'' \rrbracket_N \}$$

in a single volume. The double-stranded nucleic acids can be subjected to conditions to de-hybridize (e.g. by melting) and then to conditions promoting re-hybridization to form staggered hybridization products $\{A_1, A_2, A_3 \ldots A_N\}$ as described above. Similarly, double-stranded library fragments

$$\{ \llbracket B' \rrbracket_1, \llbracket B' \rrbracket_2, \llbracket B' \rrbracket_3 \ldots \llbracket B' \rrbracket_N \}$$

can be combined with double stranded library fragments

$$\{ \llbracket B'' \rrbracket_1, \llbracket B'' \rrbracket_2, \llbracket B'' \rrbracket_3 \ldots \llbracket B'' \rrbracket_N \}$$

in a single volume and then de-hybridized (e.g. by melting) and re-hybridized to form staggered hybridization products $\{B_1, B_2, B_3 \ldots B_N\}$ etc.

FIG. 4A shows a specific example in which two fragment A staggered hybridization products $\{A_1, A_2\}$, four fragment B staggered hybridization products $\{B_1, B_2, B_3, B_4\}$, and two fragment C staggered hybridization products $\{C_1, C_2\}$ are combined to form a non-random library of nucleic acids.

The upstream single-stranded overhang sequences of staggered hybridization products A (sequences of all of the right end) are designed to be the same as each other and to be complementary (and capable to hybridize) to the downstream single-stranded overhang sequences of staggered hybridization products B (sequences of all of the left end) which in turn are all designed to be identical. Similarly, the upstream single-stranded overhang sequences of staggered hybridization products B (sequences of all of the right end) are designed to be the same as each other and to be complementary to and to hybridize to the downstream single-stranded overhang sequences of staggered hybridization products C (sequences of all of the left end )which are all designed to be identical.

Referring to FIG. 4B, these sets of staggered hybridization products A, B, C may then be ligated in a single reaction volume to form the 16 (=2*4*2) variants $\{A_1 B_1 C_1, A_1 B_1 C_2, A_1 B_1 C_3....A_2 B_4 C_2\}$.

In some embodiments, the total number of members of the variant library is equal to the product of the number of variants of each fragment A, B, C etc. In practice, ligation reactions can be efficient for $2^{\sim 10}$ fragments being ligated. In an exemplary embodiments, 10 fragments (A,B,C...J), each with 4 variants would produce a variant library of 410 ~ 1 Million members.

In some embodiments, the fragments can have a size of about 20 bp, of about 30 bp, of about 40 bp, of about 50 bp, of about 60 bp, of about 70 bp, of about 80 bp, of about 90 bp, of about 100 bp or higher. Yet in some embodiments, the fragments can have a size of about 200 bp, of about 300 bp, of about 400 bp, of about 500 bp, of about 600 bp, of about 700 bp, of about 800 bp, of about 900 bp, of about 1000 bp, of about 2000 bp, of about 3000 bp or higher.

It should be appreciated that if fragments A, B, C etc. are the size of an oligonucleotide (~20bp to 200bp) then the library product resulting from the assembly of 10 fragments may be in the size range of individual genes (~200 bp to 2 Kbp). Such variant libraries, in which each of the members can be a variant of a gene, may be highly useful for the optimization of proteins of interest. For example, the libraries of variants may be useful for the optimization of antibodies (e.g. antibodies having specific or improved binding properties). In some embodiments, screening can be efficiently accomplished by the use of phage or yeast display or any appropriate methods known in the art. Products of interest can be reverse sequenced to find the identity of library members which have the desired properties (e.g. binding properties).

It should also appreciated that if the fragments A, B, C etc. are the size of genes (e.g. 500 bp to 2.5 Kbp, including promoters and ribosomal binding sites (RBS)) then the library products may result in a metabolic pathway. As such, the variant library may result in a library of metabolic pathway variants. In some embodiments, for a metabolic pathway having M nucleic acids comprising promoters or ribosome binding sites and proteins encoding genes, the M enzymes can each be optimized such that the catalytic output product from each enzyme reaction is matched to the input of the next enzyme and such that overall output flux of metabolite is optimized. Assuming that promoters are kept constant and that 2 RBS levels is sufficient for generating enough variants to tune the metabolic pathway, this represents $2*2M$ pathways. If M = 10, then the number of required pathways is $2*2^{10} = 2,048$ pathways. If each pathway is encoded by sequences having an average length of ~ 10Kbp, the total number of pathways can be represented by about~ 20Mbp of DNA synthesis (which represents several million dollars). By using the methods described herein, variant libraries (such as Variant Libraries by Multiplex Pathway Synthesis (VL-MPS)) may potentially be built in a single reaction in which each fragment (A, B, C etc.) can represent a promoter + RBS + enzyme encoding gene and in which each pool of fragments (A, B, C etc.) has several (e.g. 2-4) variations for the strength of either promoter or RBS. Such a library may be screened by shotgun transformation of the library of pathway variants into an expression host cell. Mass spectroscopy can be used as a read out of desired metabolite production. Alternatively, cellular based sensors such as those based on transcription factors may be used to measure desired metabolite production (Ref: Chou, Howard H., and Jay D. Keasling. "Programming adaptive control to evolve increased metabolite production." Nature Communications 4 (2013)). For example, a visual signal (e.g. by promoting Green fluorescence protein) that allows cells to be sorted by flow cytometry may be produced. In some embodiments, a factor which allows such metabolite producing cells to survive a drug marker or deficient media may be produced thus selecting for the best producing metabolic pathways.

Insertion and/or deletion variant library

Insertions and/or deletions can be a powerful tool to create a variant library of unique sequences that may have desirable properties. However, one of skill in the art will appreciate that error-prone polymerase chain reaction (PCR), or nucleic acid synthesis using degenerate bases may not suffice to create insertions or deletions of a predefined sequence, also referred herein as discrete specified sequence. Substitutions can likewise be a powerful tool to create a variant library of unique sequences. According to the present disclosure, substitution(s) can be used alone, or in any combination with insertions and/or deletions. In some embodiments, a substitution may be effected by the combination of at least (1) a deletion of 1, 2, 3 or more nucleotides, and (2) an insertion of the same number of nucleotides made at the same location in a coding region of a nucleic acid sequence. In some embodiments, substitution(s) can be a multiple of 3 consecutive nucleotides substitutions, or can encompass nucleotides in any number, including without limitation, one nucleotide, or two nucleotides, or more than two nucleotides.

Error prone PCR is a well-established method for introducing variations into a population of DNA sequences in which an error-prone polymerase creates errors as it amplifies the DNA. However, this method results in variants occurring at random positions and does not allow for the design of particular sequence that would exclude unwanted variants. Similarly, synthesis of DNA with degenerate bases is carried out when the variants are determined by indicating a degenerate base at particular positions resulting in the addition of any of the possible nucleotides at that position. During synthesis a nucleotide can be chosen from the pool of possible nucleotides at random. Because the next degenerate base relative to the previous randomly selected nucleotide is not controlled, this method does not allow for the exclusion or inclusion of particular strings of sequence, such as unwanted codons or longer fragments of relevant sequences. As such, neither of these methods allow for insertion or deletion of particular bases at predefined positions.

In some disclosure herein, nucleic acid synthesis and assembly of exact predefined sequences can be uniquely suited to produce a library of genetic material including insertions and/or deletions. In some embodiments, the method allows for the production of libraries that contain few to no extraneous sequence variants of the target nucleic acids having predefined sequences. In some embodiments, methods to synthesize nucleic acids having nucleic acid sequence insertions and/or nucleic acid sequence deletions at either an individual base level, at a codon level or at longer nucleotides sequence level are provided. In some embodiments, the methods can use nucleic acid synthesis methodologies, such as DNA synthesis, to allow for user specified sequences that include insertions and/or deletions of sections of DNA at either an individual base, a codon level or at larger portions of a nucleic acid sequence. Referring to FIG. 5, discrete sequences with deletion(s) and/or insertion(s) at the codon level (e.g. SEQ ID NO: 17, SEQ ID NO: 18, SEQ ID NO: 19), nucleotide level (e.g. SEQ ID NO: 20, SEQ ID NO: 21, SEQ ID NO: 22) and multiple nucleotide level (e.g. SEQ ID NO: 23, SEQ ID NO: 24, SEQ ID NO: 25) are synthesized. Each specific sequence is parsed such that the oligonucleotides can be synthesized separately and assembled into full variant constructs with the exact sequences as specified by the user (see FIG. 5, SEQ ID NO: 26 and SEQ ID NO: 27). Still referring to FIG. 5, discrete sequences with deletion(s) and/or insertion(s) at the codon, nucleotide and multiple nucleotide levels were synthesized and assembled. Discrete sequences with deletion(s) and/or insertion(s) at the codon level were synthesized: oligo 1, oligo 1a with deletion of nucleotide CTG and insertion of nucleotides CCG (underlined), oligo 1b with insertion of CTG, CCG (underlined) and CCG (underlined)). Discrete sequences with deletion(s) and/or insertion(s) at the nucleotide level were synthesized: oligo 2, oligo 2a with a single nucleotide deletion, oligo 2b with a single nucleotide A insertion (underlined). Discrete sequences with deletion(s) and/or insertion(s) at the multiple nucleotide level were synthetized: oligo 3, oligo 3a with 12 nucleotides deletion (underlined), oligo 3b with 12 nucleotides insertion (underlined). The oligonucleotides can be assembled into full variant constructs with the exact sequences as specified by the user: Variant 1: oligo 1 + oligo 2 + oligo 3a having the 12 nucleotides deletion and Variant 2: oligo 1a having the 3 nucleotide deletion and the 3 nucleotide insertion + oligo 2a having single nucleotide deletion + oligo 3a having the 12 nucleotides deletion. In some other embodiments, discrete sequences with deletion(s) and/or insertion(s) at the multiple nucleotide level can comprise deletions and/or insertions that are not multiple of 3 nucleotides, for example, 13 nucleotides deletions and/or insertions.

The chemistry of nucleic acid synthesis, such as deoxypolynucleotide synthesis, is a well-established process. Recently, the length of the sequence that can be synthesized has grown longer while cost of synthesis has come down. In addition, new assembly methods allow for the construction of multiple contiguous synthesis products to be formed into relevant modules for synthetic biology such as genes, small genetic networks, and even genomes. Having enabled production of this genetic material, nucleic acid synthesis can, in some embodiments, be leveraged to produce many unique variants of individual sequences. Such sequences can be used to generate, for example, pharmaceutical and chemical producers or can be used in academic research.

Highly diverse libraries of individual sequences of nucleic acids (such as DNA) can be mined through a relevant screen, and/or selection, to find the individual members of the library that have desirable properties for the intended use. Accordingly, a relatively smaller library may be used to screen or select for a function or structure of interest. In some embodiments, the libraries of variants have a high number of potentially useful amino acid substitutions at a predetermined number of positions, or potentially useful amino acid substitutions at more positions, or a combination thereof.

In some embodiments, in order to create distinct and controlled sequence content containing insertions and/or deletions, each discrete, unique sequence can be synthesized and assembled separately. In some embodiments, various combinations of specially designed construction oligonucleotides can be used. The term "construction oligonucleotide" as used herein refers to a single or double stranded oligonucleotide that may be used for assembling nucleic acid molecules that are longer than the construction oligonucleotide itself. Construction oligonucleotides may be used for assembling a nucleic acid molecule by the methods described herein. The term "polynucleotide construct" refers to a nucleic acid molecule having a longer predetermined sequence than the construction oligonucleotides. Polynucleotide constructs may be assembled from a set of construction oligonucleotides and/or a set of subassemblies.

In some embodiments, a reference sequence, with variants indicated, can first be broken up or parsed into smaller oligonucleotides that are within the range of length that can be synthesized. Some oligonucleotides can be variant oligonucleotides that include inserted or deleted bases when compared to the original "wild type" sequence. All possible oligonucleotides with deletions, insertions, variations, combinations thereof or no change can be synthesized, making up parts of the overall desired sequence(s). In some embodiments, the inclusion of variant oligonucleotides that are to be assembled requires that the sequences be parsed in such a way as to avoid variations near the junctions at which the oligonucleotides are to be assembled. Individual oligonucleotides making up all parts of the overall larger sequence can then be synthesized. These variant sequences can be assembled combinatorially resulting in all possible variants of the construct sequence including insertions and/or deletions.

According to some embodiments, the method can allow for every specific sequence to be constructed from oligonucleotide sections with each specified variant in an oligonucleotide synthesized individually. Upon assembly, every nucleic acid sequence (e.g. full construct or sub-assembly construct) may only contain variants that were explicitly indicated and as such, fewer to no extraneous variants of the construct will be created through combinatorics.

Accordingly, disclosures herein are particularly useful to produce libraries that contain large numbers of specified sequence variants. Some disclosures herein relate to libraries having large numbers of specified sequence variants and fewer or no extraneous variants of specified sequences. Libraries of the disclosure can be used to selectively screen or analyze large numbers of different predetermined nucleic acids and/or different peptides encoded by the nucleic acids.

In some embodiments, the methods of the present disclosure allow for nucleic acid libraries, such as DNA libraries, to encode variant sequences with deletions and/or insertions. In some embodiments, the insertion(s) can be a multiple of 3 nucleotides. In some embodiments, the deletion(s) can be a multiple of 3 nucleotides. In some embodiments, the insertion(s) can comprise 5 or fewer multiples of 3 nucleotides. In some embodiments, the insertion(s) can comprise 6 or fewer, 7 or fewer, 8 or fewer, 9 or fewer, 10 or fewer, 11 or fewer, 12 or fewer, or more multiples of 3 nucleotides. In some embodiments, the deletion(s) can comprise 5 or fewer multiples of 3 nucleotides. In some embodiments, the deletion(s) can comprise 6 or fewer, 7 or fewer, 8 or fewer, 9 or fewer, 10 or fewer, 11 or fewer, 12 or fewer, or more multiples of 3 nucleotides. Yet in some embodiments, the insertion(s) or deletion(s) are not multiple of 3 nucleotides. Such libraries can allow for novel protein modifications. In some embodiments, the methods of the present disclosure allow for nucleic acid libraries to encode variant sequences with large deletions and/or large insertions. Such libraries can allow for, for example, loop-in or loop-out of nucleic acids sequences encoding one or more protein domain(s) or parts of protein domains.

Disclosure herein involve combining and assembling one or more (e.g., 1, 2, 3, 4, 5, 6, 7, 8, 9, 10, or more) pools of construction oligonucleotide variants and one or more pools of construction oligonucleotide variant or invariant sequences, each pool corresponding to a different region of a target library. Each pool contains nucleic acids sequences that were selected for a region of the target nucleic acid. Accordingly, disclosures herein are particularly useful to produce libraries that contain large numbers of predefined sequence variants.

According to some disclosure herein, the method of generating a nucleic acid library comprises the steps of identifying a target nucleic acid, identifying in the target nucleic acid a first region, wherein the first region comprises a variant nucleic acid sequence; and identifying in the target nucleic acid a second region, wherein the second region comprises an invariant sequence. In some embodiments, the target nucleic acid can comprise one or more constant regions, one or more variable regions and a combination thereof. As used herein, the terms "constant", "invariant" and "non-variable" sequences are used interchangeably.

The target nucleic acid can then be parsed in at least a first plurality of oligonucleotides comprising the variant nucleic acid sequence and at least a second plurality of oligonucleotides comprising the invariant nucleic acid sequence. The at least first and second pluralities of oligonucleotides can be provided, for example synthesized, and assembled. In some embodiments, the library can be assembled using a polymerase-based assembly reaction, ligase-based assembly reaction, or a combination thereof.

In some embodiments, the target nucleic acid can encode for a polypeptide having one or more domains. In some embodiments, the variant nucleic acid sequence can comprise a deletion of nucleic acid sequences encoding at least part of the one or more domains, an insertion of nucleic acid sequences encoding at least part of the one or more domains or a combination thereof. In some embodiments, the deletion(s) and/or the insertion(s) can be a multiple of 3 nucleotides. In some embodiments, the deletion(s) and/or the insertion(s) can comprise five or fewer multiples of 3 nucleotides. In some embodiments, the deletion(s) and/or the insertion(s) can comprise 6 or fewer, 7 or fewer, 8 or fewer, 10 or fewer, 11 or fewer, 11 or fewer, 12 or fewer, or more multiples of 3 nucleotides.

In some embodiments, the insertion(s) and/or deletion(s) can be in a non-coding region of the nucleic acid, for example in the non-coding regulatory elements of a gene. For example, the insertion(s) and/or deletion(s) can be a non-coding sequence. In some embodiments, the deletion(s) and/or the insertion(s) can be single nucleotide, 2, 3, 4, 5, 6, 7, 8, 9, 10, 11, 12, 13, 14, 15, 16, 17, 18, 19, 20 or more nucleotides. In some embodiments, the deletion(s) and/or the insertion(s) can be more than 20, more than 25, more than 30, more than 35, more than 40, more than 45, more than 50, more than 55, more than 60 nucleotides.

In some embodiments, the method for producing a library of nucleic acids comprises selecting a target nucleic acid sequence, selecting at least a nucleic acid sequence to be deleted or inserted at one or more selected positions, designing a first set of oligonucleotides having variant sequences at the selected positions and at least a second set of oligonucleotides having an invariant sequence, and assembling the first and the at least second sets of oligonucleotides. In some embodiments, in the step of selecting, the nucleic acid sequence to be deleted or inserted can be a multiple of 3 nucleotides. In some embodiments, in the step of selecting, the nucleic acid sequence to be deleted or inserted can comprise five or fewer multiples of 3 nucleotides. In some embodiments, in the step of selecting, the nucleic acid sequence to be deleted or inserted can comprise 6 or fewer, 7 or fewer, 8 or fewer, 9 or fewer, 10 or fewer, 11 or fewer, 12 or fewer, or more multiples of 3 nucleotides. In some embodiments, the first and second sets together can comprise the target nucleic acid sequence. In some embodiments, the first and second sets together can comprise a fragment of the target nucleic acid sequence. In some embodiments, the selected positions can comprise a nucleotide, a codon, a sequence of nucleotides or a combination thereof.

Single Stranded Overhangs

In certain embodiments, the overlapping complementary regions between adjacent nucleic acid fragments are designed (or selected) to be sufficiently different to promote (e.g., thermodynamically favor) assembly of a unique alignment of nucleic acid fragments (e.g., a selected or designed alignment of fragments). For example, the overlapping complementary regions between adjacent nucleic acid fragments can be designed or selected to sufficiently thermodynamically favor assembly of a unique alignment of nucleic acid fragments (e.g., a selected or designed alignment of fragments). Surprisingly, under proper ligation conditions, difference by as little as one nucleotide affords sufficient discrimination power between perfect match (100% complementary cohesive ends) and mismatch (less than 100% complementary cohesive ends). As such, 4-base overhangs can allow up to (4^4+1)=257 different fragments to be ligated with high specificity and fidelity.

It should be appreciated that overlapping regions of different lengths may be used. In some embodiments, longer cohesive ends may be used when higher numbers of nucleic acid fragments are being assembled. Longer cohesive ends may provide more flexibility to design or select sufficiently distinct sequences to discriminate between correct cohesive end annealing (e.g., involving cohesive ends designed to anneal to each other) and incorrect cohesive end annealing (e.g., between non-complementary cohesive ends).

To achieve such high fidelity assembly, one or more suitable ligases may be used. A ligase may be obtained from recombinant or natural sources. In some embodiments, T3 DNA ligase, T4 DNA ligase, T7 DNA ligase, and/or *E. coli* DNA Ligase may be used. These ligases may be used at relatively low temperature (e.g., room temperature) and may be particularly useful for relatively short overhangs (e.g., about 3, about 4, about 5, or about 6 base overhangs). In certain ligation reactions (e.g., 30 min incubation at room temperature), T7 DNA ligase can be more efficient for multi-way ligation than the other ligases. A heat-stable ligase may also be used, such as one or more of Tth DNA ligase; Pfu DNA ligase; Taq ligase, any other suitable heat-stable ligase, or any combination thereof.

In some embodiments, two or more pairs of complementary cohesive ends between different nucleic acid fragments may be designed or selected to have identical or similar sequences in order to promote the assembly of products containing a relatively random arrangement (and/or number) of the fragments that have similar or identical cohesive ends. This may be useful to generate libraries of nucleic acid products with different sequence arrangements and/or different copy numbers of certain internal sequence regions.

It should be noted that to ensure ligation specificity, the overhangs can be selected or designed to be unique for each ligation site; that is, each pair of complementary overhangs for two fragments designed to be adjacent in an assembled product should be unique and differ from any other pair of complementary overhangs by at least one nucleotide.

Other methods for generating cohesive ends can also be used. For example, a polymerase based method (e.g., T4 DNA polymerase) can be used to synthesize desirable cohesive ends. Regardless of the method of generating specific overhangs (e.g., complementary overhangs for nucleic acids designed to be adjacent in an assembled nucleic acid product), overhangs of different lengths may be designed and/or produced. In some embodiments, long single-stranded overhangs (3' or 5') may be used to promote specificity and/or efficient assembly. For example, a 3' or 5' single-stranded overhang may be longer than 8 bases long, e.g., 8-14, 14-20, 20-25, 25-50, 50-100, 100-500, or more bases long.

In some embodiments, the overhangs can be from 1 to 4 bases long, from 5-12 bases long, from 1-12 bases long, from 5-13 bases long, from 6-12 bases long. In some embodiments, the overhangs can be up to 12, up to 13, up to 14, up to 15, up to 16, up to 17, up to 18, up to 19, up to 20 bases long.

In some embodiments, the overhangs can be generated by Type IIS restriction enzymes. For example, the overhangs can be from 1 to 4 bases long, or longer. A wide variety of restriction endonucleases having specific binding and/or cleavage sites are commercially available, for example, from New England Biolabs (Beverly, Mass.). In various embodiments, restriction endonucleases that produce 3' overhangs, 5' overhangs may be used. In some embodiments, sticky ends formed by the specific restriction endonuclease may be used to facilitate assembly of subassemblies in a desired arrangement. The term "type-IIs restriction endonuclease" refers to a restriction endonuclease having a non-palindromic recognition sequence and a cleavage site that occurs outside of the recognition site (e.g., from 0 to about 20 nucleotides distal to the recognition site). Type IIs restriction endonucleases may create a nick in a double-stranded nucleic acid molecule or may create a double-stranded break that produces either blunt or sticky ends (e.g., either 5' or 3' overhangs). Examples of Type IIs endonucleases include, for example, enzymes that produce a 3' overhang, such as, for example, but not limited to, Bsr I, Bsm I, BstF5 I, BsrD I, Bts I, Mnl I, BciV I, Hph I, Mbo II, Eci I, Acu I, Bpm I, Mme I, BsaX I, Bcg I, Bae I, Bfi I, TspDT I, TspGW I, Taq II, Eco57 I, Eco57M I, Gsu I, Ppi I, and Psr I; enzymes that produce a 5' overhang such as, for example, BsmA I, Ple I, Fau I, Sap I, BspM I, SfaN I, Hga I, Bvb I, Fok I, BceA I, BsmF I, Ksp632 I, Eco31 I, Esp3 I, Aar I; and enzymes that produce a blunt end, such as, for example, Mly I and Btr I. Type-IIs endonucleases are commercially available and are well known in the art (New England Biolabs, Beverly, Mass.).

In some embodiments, the overhangs can be designed such that they have minimal self-complementarity. For example, the overhangs can be designed to be from 5 to 12 bases long and with a minimal tendency to form hairpins. Yet in other embodiments, the overhangs can be designed to have self-complementarity. For example, the overhangs can be designed to be from 3 to 12 bases long with a tendency to form hairpins.

High Fidelity Assembly

According to disclosure herein, a plurality of nucleic acid fragments may be assembled in a single procedure wherein the plurality of fragments is mixed together under conditions that promote covalent assembly of the fragments to generate a specific longer nucleic acid. According to disclosure herein, a plurality of nucleic acid fragments may be covalently assembled in vitro using a ligase. In some embodiments, 5 or more (e.g., 10 or more, 15 or more, 15 to 20, 20 to 25, 25 to 30, 30 to 35, 35 to 40, 40 to 45, 45 to 50, 50 or more, etc.) different nucleic acid fragments may be assembled. However, it should be appreciated that any number of nucleic acids (e.g., 2, 3, 4, 5, 6, 7, 8, 9, 10, 11, 12, 13, 14, 15, 16, 17, 18, 19, 20, etc.) may be assembled using suitable assembly techniques. Each nucleic acid fragment being assembled may be between about 100 nucleotides long and about 1,000 nucleotides long (e.g., about 200, about 300, about 400, about 500, about 600, about 700, about 800, about 900). However, longer (e.g., about 2,500 or more nucleotides long, about 5,000 or more nucleotides long, about 7,500 or more nucleotides long, about 10,000 or more nucleotides long, etc.) or shorter nucleic acid fragments may be assembled using an assembly technique (e.g., shotgun assembly into a plasmid vector). It should be appreciated that the size of each nucleic acid fragment may be independent of the size of other nucleic acid fragments added to an assembly. However, in some embodiments, each nucleic acid fragment may be approximately the same size or length (e.g., between about 100 nucleotides long and about 400 nucleotides long). For example, the length of the oligonucleotides may have a median length of between about 100 nucleotides long and about 400 nucleotides long and vary from about, +/- 1 nucleotides, +/- 4 nucleotides, +/- 10 nucleotides. It should be appreciated that the length of a double-stranded nucleic acid fragment may be indicated by the number of base pairs. As used herein, a nucleic acid fragment referred to as "x" nucleotides long corresponds to "x" base pairs in length when used in the context of a double-stranded nucleic acid fragment. In some embodiments, one or more nucleic acids being assembled in one reaction (e.g., 1-5, 5-10, 10-15, 15-20, etc.) may be codon-optimized and/or non-naturally occurring. In some embodiments, all of the nucleic acids being assembled in one reaction are codon-optimized and/or non-naturally occurring.

In some disclosure herein, nucleic acid fragments being assembled are designed to have overlapping complementary sequences. In some embodiments, the nucleic acid fragments are double-stranded nucleic acid fragments with 3' and/or 5' single-stranded overhangs. These overhangs may be cohesive ends that can anneal to complementary cohesive ends on different nucleic acid fragments. According to disclosure herein, the presence of complementary sequences (and particularly complementary cohesive ends) on two nucleic acid fragments promotes their covalent assembly. In some embodiments, a plurality of nucleic acid fragments with different overlapping complementary single-stranded cohesive ends is assembled and their order in the assembled nucleic acid product is determined by the identity of the cohesive ends on each fragment. For example, the nucleic acid fragments may be designed so that a first nucleic acid has a first cohesive end that is complementary to a first cohesive end of a second nucleic acid and a second cohesive end that is complementary to a first cohesive end of a third nucleic acid. A second cohesive end of the second nucleic acid may be complementary to a first cohesive end of a fourth nucleic acid. A second cohesive end of the third nucleic acid may be complementary a first cohesive end of a fifth nucleic acid. And so on through to the final nucleic acid. According to disclosure herein, this technique may be used to generate a linear arrangement containing nucleic acid fragments assembled in a predetermined linear order (e.g., first, second, third, fourth, ..., final).

In certain embodiments, the overlapping complementary regions between adjacent nucleic acid fragments are designed (or selected) to be sufficiently different to promote (e.g., thermodynamically favor) assembly of a unique alignment of nucleic acid fragments (e.g., a selected or designed alignment of fragments). Surprisingly, under proper ligation conditions, difference by as little as one nucleotide affords sufficient discrimination power between perfect match (100% complementary cohesive ends) and mismatch (less than 100% complementary cohesive ends). As such, 4-base overhangs can theoretically allow up to (4^4+1)=257 different fragments to be ligated with high specificity and fidelity.

It should be appreciated that overlapping regions of different lengths may be used. In some embodiments, longer cohesive ends may be used when higher numbers of nucleic acid fragments are being assembled. Longer cohesive ends may provide more flexibility to design or select sufficiently distinct sequences to discriminate between correct cohesive end annealing (e.g., involving cohesive ends designed to anneal to each other) and incorrect cohesive end annealing (e.g., between non-complementary cohesive ends).

To achieve such high fidelity assembly, one or more suitable ligases may be used. A ligase may be obtained from recombinant or natural sources. In some embodiments, T3 DNA ligase, T4 DNA ligase, T7 DNA ligase, and/or *E. coli* DNA Ligase may be used. These ligases may be used at relatively low temperature (e.g., room temperature) and particularly useful for relatively short overhangs (e.g., about 3, about 4, about 5, or about 6 base overhangs). In certain ligation reactions (e.g., 30 min incubation at room temperature), T7 DNA ligase can be more efficient for multi-way ligation than the other ligases. A heat-stable ligase may also be used, such as one or more of Tth DNA ligase; Pfu DNA ligase; Taq ligase, any other suitable heat-stable ligase, or any combination thereof.

In some embodiments, two or more pairs of complementary cohesive ends between different nucleic acid fragments may be designed or selected to have identical or similar sequences in order to promote the assembly of products containing a relatively random arrangement (and/or number) of the fragments that have similar or identical cohesive ends. This may be useful to generate libraries of nucleic acid products with different sequence arrangements and/or different copy numbers of certain internal sequence regions.

In some embodiments, the nucleic acid fragments are mixed and incubated with a ligase. It should be appreciated that incubation under conditions that promote specific annealing of the cohesive ends may increase the frequency of assembly (e.g., correct assembly). In some embodiments, the different cohesive ends are designed to have similar melting temperatures (e.g., within about 5 °C of each other) so that correct annealing of all of the fragments is promoted under the same conditions. Correct annealing may be promoted at a different temperature depending on the length of the cohesive ends that are used. In some embodiments, cohesive ends of between about 4 and about 30 nucleotides in length (e.g., cohesive ends of about 5, about 10, about 15, about 20, about 25, or about 30 nucleotides in length) may be used. Incubation temperatures may range from about 20°C to about 50°C (including, e.g., room temperature). However, higher or lower temperatures may be used. The length of the incubation may be optimized based on the length of the overhangs, the complexity of the overhangs, and the number of different nucleic acids (and therefore the number of different overhangs) that are mixed together. The incubation time also may depend on the annealing temperature and the presence or absence of other agents in the mixture. For example, a nucleic acid binding protein and/or a recombinase may be added (e.g., RecA, for example a heat stable RecA protein).

The resulting complex of nucleic acids may be subjected to a polymerase chain reaction, in the presence of a pair of target-sequence specific primers, to amplify and select for the correct ligation product (i.e., the target nucleic acid). Alternatively, the resulting complex of nucleic acids can be ligated into a suitable vector and transformed into a host cell for further colony screening.

Support

As used herein, the terms "support" and "substrate" are used interchangeably and refer to a porous or non-porous solvent insoluble material on which polymers such as nucleic acids are synthesized or immobilized. As used herein "porous" means that the material contains pores having substantially uniform diameters (for example in the nm range). Porous materials can include but are not limited to, paper, synthetic filters and the like. In such porous materials, the reaction may take place within the pores. The support can have any one of a number of shapes, such as pin, strip, plate, disk, rod, bends, cylindrical structure, particle, including bead, nanoparticle and the like. The support can have variable widths.

The support can be hydrophilic or capable of being rendered hydrophilic. The support can include inorganic powders such as silica, magnesium sulfate, and alumina; natural polymeric materials, particularly cellulosic materials and materials derived from cellulose, such as fiber containing papers, e.g., filter paper, chromatographic paper, etc.; synthetic or modified naturally occurring polymers, such as nitrocellulose, cellulose acetate, poly (vinyl chloride), polyacrylamide, cross linked dextran, agarose, polyacrylate, polyethylene, polypropylene, poly (4-methylbutene), polystyrene, polymethacrylate, poly(ethylene terephthalate), nylon, poly(vinyl butyrate), polyvinylidene difluoride (PVDF) membrane, glass, controlled pore glass, magnetic controlled pore glass, ceramics, metals, and the like; either used by themselves or in conjunction with other materials.

In some embodiments, oligonucleotides are synthesized on an array format. For example, single-stranded oligonucleotides are synthesized in situ on a common support wherein each oligonucleotide is synthesized on a separate or discrete feature (or spot) on the substrate. In preferred embodiments, single-stranded oligonucleotides are bound to the surface of the support or feature. As used herein, the term "array" refers to an arrangement of discrete features for storing, routing, amplifying and releasing oligonucleotides or complementary oligonucleotides for further reactions. In a preferred embodiment, the support or array is addressable: the support includes two or more discrete addressable features at a particular predetermined location (i.e., an "address") on the support. Therefore, each oligonucleotide molecule of the array is localized to a known and defined location on the support. The sequence of each oligonucleotide can be determined from its position on the support. Moreover, addressable supports or arrays enable the direct control of individual isolated volumes such as droplets. The size of the defined feature can be chosen to allow formation of a microvolume droplet on the feature, each droplet being kept separate from each other. As described herein, features are typically, but need not be, separated by interfeature spaces to ensure that droplets between two adjacent features do not merge. Interfeatures will typically not carry any oligonucleotide on their surface and will correspond to inert space. In some embodiments, features and interfeatures may differ in their hydrophilicity or hydrophobicity properties. In some embodiments, features and interfeatures may comprise a modifier as described herein.

Arrays may be constructed, custom ordered or purchased from a commercial vendor (e.g., CombiMatrix, Agilent, Affymetrix, Nimblegen). Oligonucleotides are attached, spotted, immobilized, surface-bound, supported or synthesized on the discrete features of the surface or array. Oligonucleotides may be covalently attached to the surface or deposited on the surface. Various methods of construction are well known in the art, e.g., maskless array synthesizers, light directed methods utilizing masks, flow channel methods, spotting methods etc.

In other embodiments, a plurality of oligonucleotides may be synthesized or immobilized (e.g., attached) on multiple supports, such as beads. One example is a bead based synthesis method which is described, for example, in U.S. Pat. Nos. 5,770,358; 5,639,603; and 5,541,061. For the synthesis of molecules such as oligonucleotides on beads, a large plurality of beads is suspended in a suitable carrier (such as water) in a container. The beads are provided with optional spacer molecules having an active site to which is complexed, optionally, a protecting group. At each step of the synthesis, the beads are divided for coupling into a plurality of containers. After the nascent oligonucleotide chains are deprotected, a different monomer solution is added to each container, so that on all beads in a given container, the same nucleotide addition reaction occurs. The beads are then washed of excess reagents, pooled in a single container, mixed and re-distributed into another plurality of containers in preparation for the next round of synthesis. It should be noted that by virtue of the large number of beads utilized at the outset, there will similarly be a large number of beads randomly dispersed in the container, each having a unique oligonucleotide sequence synthesized on a surface thereof after numerous rounds of randomized addition of bases. An individual bead may be tagged with a sequence which is unique to the double-stranded oligonucleotide thereon, to allow for identification during use.

In yet another embodiment, a plurality of oligonucleotides may be attached or synthesized on nanoparticles. Nanoparticles include but are not limited to metal (e.g., gold, silver, copper and platinum), semiconductor (e.g., CdSe, CdS, and CdS coated with ZnS) and magnetic (e.g., ferromagnetite) colloidal materials. Methods to attach oligonucleotides to the nanoparticles are known in the art. In another embodiment, nanoparticles are attached to the substrate. Nanoparticles with or without immobilized oligonucleotides can be attached to substrates as described in, e.g., Grabar et al., Analyt. Chem., 67, 73-743 (1995); Bethell et al., J. Electroanal. Chem., 409, 137 (1996); Bar et al., Langmuir, 12, 1172 (1996); Colvin et al., J. Am. Chem. Soc., 114, 5221 (1992). Naked nanoparticles may be first attached to the substrate and oligonucleotides can be attached to the immobilized nanoparticles.

Pre-synthesized oligonucleotide and/or polynucleotide sequences may be attached to a support or synthesized in situ using light-directed methods, flow channel and spotting methods, inkjet methods, pin-based methods and bead-based methods known in the art In some embodiments, pre-synthesized oligonucleotides are attached to a support or are synthesized using a spotting methodology wherein monomers solutions are deposited dropwise by a dispenser that moves from region to region (e.g., ink jet). In some embodiments, oligonucleotides are spotted on a support using, for example, a mechanical wave actuated dispenser.

Applications

Disclosure herein may be useful for a range of applications involving the production and/or use of synthetic nucleic acids. As described herein, the disclosure provides methods for assembling synthetic nucleic acids with increased efficiency. The resulting assembled nucleic acids may be amplified in vitro (e.g., using PCR, LCR, or any suitable amplification technique), amplified in vivo (e.g., via cloning into a suitable vector), isolated and/or purified. An assembled nucleic acid (alone or cloned into a vector) may be transformed into a host cell (e.g., a prokaryotic, eukaryotic, insect, mammalian, or other host cell). In some embodiments, the host cell may be used to propagate the nucleic acid. In certain embodiments, the nucleic acid may be integrated into the genome of the host cell. In some embodiments, the nucleic acid may replace a corresponding nucleic acid region on the genome of the cell (e.g., via homologous recombination). Accordingly, nucleic acids may be used to produce recombinant organisms. In some embodiments, a target nucleic acid may be an entire genome or large fragments of a genome that are used to replace all or part of the genome of a host organism. Recombinant organisms also may be used for a variety of research, industrial, agricultural, and/or medical applications.

Many of the techniques described herein can be used together, applying suitable assembly techniques at one or more points to produce long nucleic acid molecules. For example, ligase-based assembly may be used to assemble oligonucleotide duplexes and nucleic acid fragments of less than 100 to more than 10,000 base pairs in length (e.g., 100 mers to 500 mers, 500 mers to 1,000 mers, 1,000 mers to 5,000 mers, 5, 000 mers to 10,000 mers, 25,000 mers, 50,000 mers, 75,000 mers, 100,000 mers, etc.). In an exemplary embodiment, methods described herein may be used during the assembly of an entire genome (or a large fragment thereof, e.g., about 10%, 20%, 30%, 40%, 50%, 60%, 70%, 80%, 90%, or more) of an organism (e.g., of a viral, bacterial, yeast, or other prokaryotic or eukaryotic organism), optionally incorporating specific modifications into the sequence at one or more desired locations.

Any of the nucleic acid products (e.g., including nucleic acids that are amplified, cloned, purified, isolated, etc.) may be packaged in any suitable format (e.g., in a stable buffer, lyophilized, etc.) for storage and/or shipping (e.g., for shipping to a distribution center or to a customer). Similarly, any of the host cells (e.g., cells transformed with a vector or having a modified genome) may be prepared in a suitable buffer for storage and or transport (e.g., for distribution to a customer). In some embodiments, cells may be frozen. However, other stable cell preparations also may be used.

Host cells may be grown and expanded in culture. Host cells may be used for expressing one or more RNAs or polypeptides of interest (e.g., therapeutic, industrial, agricultural, and/or medical proteins). The expressed polypeptides may be natural polypeptides or non-natural polypeptides. The polypeptides may be isolated or purified for subsequent use.

Accordingly, nucleic acid molecules generated using methods of the disclosure can be incorporated into a vector. The vector may be a cloning vector or an expression vector. In some embodiments, the vector may be a viral vector. A viral vector may comprise nucleic acid sequences capable of infecting target cells. Similarly, in some embodiments, a prokaryotic expression vector operably linked to an appropriate promoter system can be used to transform target cells. In other embodiments, a eukaryotic vector operably linked to an appropriate promoter system can be used to transfect target cells or tissues.

Transcription and/or translation of the constructs described herein may be carried out in vitro (i.e. using cell-free systems) or in vivo (i.e. expressed in cells). In some embodiments, cell lysates may be prepared. In certain embodiments, expressed RNAs or polypeptides may be isolated or purified. Nucleic acids of the disclosure also may be used to add detection and/or purification tags to expressed polypeptides or fragments thereof. Examples of polypeptide-based fusion/tag include, but are not limited to, hexa-histidine (His[6]) Myc and HA, and other polypeptides with utility, such as $GFP_5$ GST, MBP, chitin and the like. In some embodiments, polypeptides may comprise one or more unnatural amino acid residue(s).

In some embodiments, antibodies can be made against polypeptides or fragment(s) thereof encoded by one or more synthetic nucleic acids. In certain embodiments, synthetic nucleic acids may be provided as libraries for screening in research and development (e.g., to identify potential therapeutic proteins or peptides, to identify potential protein targets for drug development, etc.) In some embodiments, a synthetic nucleic acid may be used as a therapeutic (e.g., for gene therapy, or for gene regulation). For example, a synthetic nucleic acid may be administered to a patient in an amount sufficient to express a therapeutic amount of a protein. In other embodiments, a synthetic nucleic acid may be administered to a patient in an amount sufficient to regulate (e.g., down-regulate) the expression of a gene.

It should be appreciated that different acts or embodiments described herein may be performed independently and may be performed at different locations in the United States or outside the United States. For example, each of the acts of receiving an order for a target nucleic acid, analyzing a target nucleic acid sequence, designing one or more starting nucleic acids (e.g., oligonucleotides), synthesizing starting nucleic acid(s), purifying starting nucleic acid(s), assembling starting nucleic acid(s), isolating assembled nucleic acid(s), confirming the sequence of assembled nucleic acid(s), manipulating assembled nucleic acid(s) (e.g., amplifying, cloning, inserting into a host genome, etc.), and any other acts or any parts of these acts may be performed independently either at one location or at different sites within the United States or outside the United States. In some embodiments, an assembly procedure may involve a combination of acts that are performed at one site (in the United States or outside the United States) and acts that are performed at one or more remote sites (within the United States or outside the United States).

Automated Applications

Aspects of the methods and devices provided herein may include automating one or more acts described herein. In some embodiments, one or more steps of an amplification and/or assembly reaction may be automated using one or more automated sample handling devices (e.g., one or more automated liquid or fluid handling devices). Automated devices and procedures may be used to deliver reaction reagents, including one or more of the following: starting nucleic acids, buffers, enzymes (e.g., one or more ligases and/or polymerases), nucleotides, salts, and any other suitable agents such as stabilizing agents. Automated devices and procedures also may be used to control the reaction conditions. For example, an automated thermal cycler may be used to control reaction temperatures and any temperature cycles that may be used. In some embodiments, a scanning laser may be automated to provide one or more reaction temperatures or temperature cycles suitable for incubating polynucleotides. Similarly, subsequent analysis of assembled polynucleotide products may be automated. For example, sequencing may be automated using a sequencing device and automated sequencing protocols. Additional steps (e.g., amplification, cloning, etc.) also may be automated using one or more appropriate devices and related protocols. It should be appreciated that one or more of the device or device components described herein may be combined in a system (e.g., a robotic system) or in a micro-environment (e.g., a micro-fluidic reaction chamber). Assembly reaction mixtures (e.g., liquid reaction samples) may be transferred from one component of the system to another using automated devices and procedures (e.g., robotic manipulation and/or transfer of samples and/or sample containers, including automated pipetting devices, micro-systems, etc.). The system and any components thereof may be controlled by a control system.

Accordingly, method steps and/or aspects of the devices provided herein may be automated using, for example, a computer system (e.g., a computer controlled system). A computer system on which aspects of the technology provided herein can be implemented may include a computer for any type of processing (e.g., sequence analysis and/or automated device control as described herein). However, it should be appreciated that certain processing steps may be provided by one or more of the automated devices that are part of the assembly system. In some embodiments, a computer system may include two or more computers. For example, one computer may be coupled, via a network, to a second computer. One computer may perform sequence analysis. The second computer may control one or more of the automated synthesis and assembly devices in the system. In other aspects, additional computers may be included in the network to control one or more of the analysis or processing acts. Each computer may include a memory and processor. The computers can take any form, as the aspects of the technology provided herein are not limited to being implemented on any particular computer platform. Similarly, the network can take any form, including a private network or a public network (e.g., the Internet). Display devices can be associated with one or more of the devices and computers. Alternatively, or in addition, a display device may be located at a remote site and connected for displaying the output of an analysis in accordance with the technology provided herein. Connections between the different components of the system may be via wire, optical fiber, wireless transmission, satellite transmission, any other suitable transmission, or any combination of two or more of the above.

Each of the different aspects, embodiments, or acts of the technology provided herein can be independently automated and implemented in any of numerous ways. For example, each aspect, embodiment, or act can be independently implemented using hardware, software or a combination thereof. When implemented in software, the software code can be executed on any suitable processor or collection of processors, whether provided in a single computer or distributed among multiple computers. It should be appreciated that any component or collection of components that perform the functions described above can be generically considered as one or more controllers that control the above-discussed functions. The one or more controllers can be implemented in numerous ways, such as with dedicated hardware, or with general purpose hardware (e.g., one or more processors) that is programmed using microcode or software to perform the functions recited above.

In this respect, it should be appreciated that one implementation of the embodiments of the technology provided herein comprises at least one computer-readable medium (e.g., a computer memory, a floppy disk, a compact disk, a tape, etc.) encoded with a computer program (i.e., a plurality of instructions), which, when executed on a processor, performs one or more of the above-discussed functions of the technology provided herein. The computer-readable medium can be transportable such that the program stored thereon can be loaded onto any computer system resource to implement one or more functions of the technology provided herein. In addition, it should be appreciated that the reference to a computer program which, when executed, performs the above-discussed functions, is not limited to an application program running on a host computer. Rather, the term computer program is used herein in a generic sense to reference any type of computer code (e.g., software or microcode) that can be employed to program a processor to implement the above-discussed aspects of the technology provided herein.

It should be appreciated that in accordance with several embodiments of the technology provided herein wherein processes are stored in a computer readable medium, the computer implemented processes may, during the course of their execution, receive input manually (e.g., from a user).

Accordingly, overall system-level control of the assembly devices or components described herein may be performed by a system controller which may provide control signals to the associated nucleic acid synthesizers, liquid handling devices, thermal cyclers, sequencing devices, associated robotic components, as well as other suitable systems for performing the desired input/output or other control functions. Thus, the system controller along with any device controllers together forms a controller that controls the operation of a nucleic acid assembly system. The controller may include a general purpose data processing system, which can be a general purpose computer, or network of general purpose computers, and other associated devices, including communications devices, modems, and/or other circuitry or components to perform the desired input/output or other functions. The controller can also be implemented, at least in part, as a single special purpose integrated circuit (e.g., ASIC) or an array of ASICs, each having a main or central processor section for overall, system-level control, and separate sections dedicated to performing various different specific computations, functions and other processes under the control of the central processor section. The controller can also be implemented using a plurality of separate dedicated programmable integrated or other electronic circuits or devices, e.g., hard wired electronic or logic circuits such as discrete element circuits or programmable logic devices. The controller can also include any other components or devices, such as user input/output devices (monitors, displays, printers, a keyboard, a user pointing device, touch screen, or other user interface, etc.), data storage devices, drive motors, linkages, valve controllers, robotic devices, vacuum and other pumps, pressure sensors, detectors, power supplies, pulse sources, communication devices or other electronic circuitry or components, and so on. The controller also may control operation of other portions of a system, such as automated client order processing, quality control, packaging, shipping, billing, etc., to perform other suitable functions known in the art but not described in detail herein.

Various aspects of the present invention may be used alone, in combination, or in a variety of arrangements

EP 3 074 554 B1

not specifically discussed in the embodiments described in the foregoing and is therefore not limited in its application to the details and arrangement of components set forth in the foregoing description or illustrated in the drawings. For example, aspects described in one embodiment may be combined in any manner with aspects described in other embodiments.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

## Claims

1. A method for generating a nucleic acid library comprising a plurality of predetermined unique non-random variant target nucleic acids, the method comprising:

   (a) providing a first plurality of partial double-stranded nucleic acids in a first volume,
   wherein each of the first plurality of partial double-stranded nucleic acids is a predetermined variant sequence,
   wherein a first strand of each partial double-stranded nucleic acid in the first plurality has identical single-stranded overhangs, and a second strand of each partial double-stranded nucleic acid in the first plurality has identical single-stranded overhangs,
   wherein each of the first plurality of partial double-stranded nucleic acids is a predetermined variant sequence that is unique in the first plurality of partial double-stranded nucleic acids;
   (b) providing a second plurality of partial double-stranded nucleic acids in a second volume,
   wherein each of the second plurality of partial double-stranded nucleic acids is a predetermined invariant sequence,
   wherein a first strand of each partial double-stranded nucleic acid in the second plurality has identical single-stranded overhangs and wherein a second strand of each partial double-stranded nucleic acid in the second plurality has identical single-stranded overhangs,
   wherein the single-stranded overhang of either the first strand or second strand of each partial double-stranded nucleic acid in the second plurality is complementary to the single stranded overhang of either the first strand or the second strand of each partial double-stranded nucleic acid in the first plurality, and
   wherein each of the second plurality of partial double-stranded nucleic acids is the same predetermined invariant sequence; and
   (c) assembling the library of nucleic acids by mixing the first plurality of partial double-stranded nucleic acids with the second plurality of partial double-stranded nucleic acids under conditions to hybridize the complementary overhangs to form the library of predetermined unique non-random variant target nucleic acids.

2. The method of claim 1 wherein, in the step of assembling, the complementary overhangs hybridize to form gapless junctions and are ligated, wherein preferably the step of assembling is performed in a single reaction volume.

3. The method of claim 1 wherein the step of providing the first and the second pluralities of partial double stranded nucleic acids comprises:

   (i) providing a first plurality of sets of blunt-ended double-stranded nucleic acids in the first volume,

      wherein a first nucleic acid of a first set of blunt-ended double-stranded nucleic acids has a sequence that is offset by n bases from a second nucleic acid of the first set of blunt-ended double-stranded nucleic acids, and
      wherein each double-stranded nucleic acid in each set of blunt-ended double-stranded nucleic acids is unique in the set;

(ii) providing a second plurality of sets of blunt-ended double-stranded nucleic acids in the second volume wherein a first nucleic acid of a second set of blunt-ended double-stranded nucleic acids has a sequence that is offset by n bases from a second nucleic acid of the second set of blunt-ended double-stranded nucleic acids;

(iii) melting the first plurality of sets of blunt-ended double-stranded nucleic acids in the first volume thereby forming single-stranded nucleic acids in the first volume and melting the second plurality of sets of blunt-ended double-stranded nucleic acids in the second volume thereby forming single-stranded nucleic acids in the second volume; and

(iv) annealing the single-stranded oligonucleotides to form the first plurality of partial double-stranded oligonucleotides in the first volume and the second plurality of partial double-stranded oligonucleotides in the second volume; wherein preferably n is 2, 3, 4, 5, 6, 7, or 8 bases.

4. The method of any one of claims 1-3 further comprising a third plurality of partial double-stranded nucleic acids in a third volume, wherein each of the third plurality of double-stranded nucleic acids has identical single-stranded overhangs, wherein each of the third plurality of partial double-stranded nucleic acids is unique in the third plurality of partial double-stranded nucleic acids; wherein preferably the method further comprises assembling the library of variant nucleic acids by mixing the first, second and third pluralities of partial double-stranded nucleic acids under conditions to hybridize the complementary overhangs to form the library of non-random variant target nucleic acids.

5. The method of claim 1 wherein the library is a library of genes or a library of metabolic pathways; wherein preferably each partial double-stranded nucleic acid has a size ranging from about 20 bases pairs to about 200 bases pairs or from about 500 bases pairs to about 3000 bases pairs.

6. The method of claim 1 wherein each partial double-stranded nucleic acid is selected from an operon comprising a promoter sequence, a ribosomal binding site sequence and a gene or set of genes and any combination thereof, wherein preferably the library is a library of operons comprising promoters having different strengths or a library of operons comprising ribosomal binding sites having different strengths.

7. The method of claim 1, the method further comprising, prior to step (a):

(i) identifying a target nucleic acid;

(ii) identifying in the target nucleic acid a first region, wherein the first region comprises a variant nucleic acid sequence;

(iii) identifying in the target nucleic acid a second region, wherein the second region comprises an invariant sequence; and

(iv) parsing the target nucleic acid in at least a first plurality of oligonucleotides comprising the variant nucleic acid sequence and at least a second plurality of oligonucleotides comprising the invariant nucleic acid sequence, wherein each of the first plurality of oligonucleotides has a sequence that is unique in the first plurality of oligonucleotides.

8. The method of claim 7 wherein the target nucleic acid encodes a polypeptide having one or more domains; wherein preferably the first plurality of oligonucleotides comprises a deletion or insertion of nucleic acid sequences encoding at least part of the one or more domains, or a combination thereof; wherein preferably the deletion or the insertion is a multiple of 3 nucleotides, more preferably five or less multiples of 3 nucleotides or up to 12 multiples of 3 nucleotides.

9. The method of claim 7 wherein the target nucleic acid comprises one or more constant regions and/or one or more variable regions.

10. The method of claim 7 wherein the library is assembled using a polymerase-based approach, a ligase-based approach, or a combination thereof.

11. The method of claim 7 wherein the target nucleic acid is a gene or a set of genes, wherein preferably the nucleic acid library comprises a deletion, an insertion or a combination thereof in the non-coding sequence of the gene or set of genes.

12. The method of claim 1, the method further comprising, prior to step (a):

(i) selecting a target nucleic acid sequence;

(ii) selecting at least a nucleic acid sequence to be deleted or inserted at one or more selected positions; and
(iii) designing a first set of oligonucleotides having variant sequences at the selected positions and at least a second set of oligonucleotides comprising an invariant sequence, wherein each of the first set of oligonucleotides has a sequence that is unique in the first set of oligonucleotides.

13. The method of claim 12 wherein the selected positions comprises a nucleotide, a codon, a sequence of nucleotides or a combination thereof.

14. The method of claim 12 wherein, in the step of selecting, the nucleic acid sequence to be deleted or inserted is a multiple of 3 nucleotides, preferably five or less multiples of 3 nucleotides or up to 12 multiples of 3 nucleotides.

15. The method of claim 12 wherein the target nucleic acid is a gene or a set of genes, wherein preferably the nucleic acid library comprises a deletion, an insertion or a combination thereof in the non-coding sequence of the gene or set of genes.

**Patentansprüche**

1. Verfahren zum Erstellen einer Nucleinsäurebibliothek, umfassend eine Vielzahl an vorbestimmten einzigartigen nichtzufälligen Zielnucleinsäurevarianten, wobei das Verfahren Folgendes umfasst:

(a) Bereitstellen einer ersten Vielzahl von partiellen doppelsträngigen Nucleinsäuren in einem ersten Volumen, wobei jede aus der ersten Vielzahl von partiellen doppelsträngigen Nucleinsäuren eine vorbestimmte Sequenzvariante ist,
wobei ein erster Strang jeder partiellen doppelsträngigen Nucleinsäure in der ersten Vielzahl identische einzelsträngige Überhänge aufweist und ein zweiter Strang jeder partiellen doppelsträngigen Nucleinsäure in der ersten Vielzahl identische einzelsträngige Überhänge aufweist,
wobei jede aus der ersten Vielzahl von partiellen doppelsträngigen Nucleinsäuren eine vorbestimmte Sequenzvariante ist, die in der ersten Vielzahl von partiellen doppelsträngigen Nucleinsäuren einzigartig ist;
(b) Bereitstellen einer zweiten Vielzahl von partiellen doppelsträngigen Nucleinsäuren in einem zweiten Volumen,
wobei jede aus der zweiten Vielzahl von partiellen doppelsträngigen Nucleinsäuren eine vorbestimmte invariante Sequenz ist,
wobei ein erster Strang jeder partiellen doppelsträngigen Nucleinsäure in der zweiten Vielzahl identische einzelsträngige Überhänge aufweist und wobei ein zweiter Strang jeder partiellen doppelsträngigen Nucleinsäure in der zweiten Vielzahl identische einzelsträngige Überhänge aufweist,
wobei der einzelsträngige Überhang entweder des ersten Strangs oder des zweiten Strangs jeder partiellen doppelsträngigen Nucleinsäure in der zweiten Vielzahl komplementär zu dem einzelsträngigen Überhang entweder des ersten Strangs oder des zweiten Strangs jeder partiellen doppelsträngigen Nucleinsäure in der ersten Vielzahl ist und
wobei jede aus der zweiten Vielzahl von partiellen doppelsträngigen Nucleinsäuren dieselbe vorbestimmte invariante Sequenz ist; und
(c) Assemblieren der Bibliothek von Nucleinsäuren durch Vermischen der ersten Vielzahl von partiellen doppelsträngigen Nucleinsäuren mit der zweiten Vielzahl von partiellen doppelsträngigen Nucleinsäuren unter Bedingungen, um die komplementären Überhänge zu hybridisieren, um die Bibliothek aus vorbestimmten einzigartigen nichtzufälligen Zielnucleinsäurevarianten zu erstellen.

2. Verfahren nach Anspruch 1, wobei in dem Schritt des Assemblierens die komplementären Überhänge hybridisieren, um lückenlose Übergänge zu bilden, und ligiert werden, wobei der Schritt des Assemblierens vorzugsweise in einem einzigen Reaktionsvolumen durchgeführt wird.

3. Verfahren nach Anspruch 1, wobei der Schritt des Bereitstellens der ersten und der zweiten Vielzahl von partiellen doppelsträngigen Nucleinsäuren Folgendes umfasst:

(i) Bereitstellen einer ersten Vielzahl von Sätzen von stumpfen doppelsträngigen Nucleinsäuren in dem ersten Volumen,
wobei eine erste Nucleinsäure eines ersten Satzes von stumpfen doppelsträngigen Nucleinsäuren eine Sequenz aufweist, die um n Basen von einer zweiten Nucleinsäure des ersten Satzes von stumpfen doppelsträngigen Nucleinsäuren versetzt ist, und

wobei jede doppelsträngige Nucleinsäure in jedem Satz von stumpfen doppelsträngigen Nucleinsäuren in dem Satz einzigartig ist;

(ii) Bereitstellen einer zweiten Vielzahl von Sätzen von stumpfen doppelsträngigen Nucleinsäuren in dem zweiten Volumen, wobei eine Nucleinsäure eines zweiten Satzes von stumpfen doppelsträngigen Nucleinsäuren eine Sequenz aufweist, die um n Basen von einer zweiten Nucleinsäure des zweiten Satzes von stumpfen doppelsträngigen Nucleinsäuren versetzt ist;

(iii) Schmelzen der ersten Vielzahl von Sätzen von stumpfen doppelsträngigen Nucleinsäuren in dem ersten Volumen, wodurch einzelsträngige Nucleinsäuren in dem ersten Volumen gebildet werden, und Schmelzen der zweiten Vielzahl von Sätzen von stumpfen doppelsträngigen Nucleinsäuren in dem zweiten Volumen, wodurch einzelsträngige Nucleinsäuren in dem zweiten Volumen gebildet werden; und

(iv) Anellieren der einzelsträngigen Oligonucleotide, um die erste Vielzahl von partiellen doppelsträngigen Oligonucleotiden in dem ersten Volumen und die zweite Vielzahl von partiellen doppelsträngigen Oligonucleotiden in dem zweiten Volumen zu bilden; wobei n vorzugsweise 2, 3, 4, 5, 6, 7 oder 8 Basen beträgt.

4. Verfahren nach einem der Ansprüche 1 bis 3, das weiters eine dritte Vielzahl von partiellen doppelsträngigen Nucleinsäuren in einem dritten Volumen umfasst, wobei jede aus der dritten Vielzahl von doppelsträngigen Nucleinsäuren identische einzelsträngige Überhänge aufweist, wobei jede aus der dritten Vielzahl von partiellen doppelsträngigen Nucleinsäuren in der dritten Vielzahl von partiellen doppelsträngigen Nucleinsäuren einzigartig ist; wobei das Verfahren vorzugsweise weiters das Assemblieren der Bibliothek von Nucleinsäurevarianten durch Vermischen der ersten, zweiten und dritten Vielzahl von partiellen doppelsträngigen Nucleinsäuren unter Bedingungen umfasst, um die komplementären Überhänge zu hybridisieren, um die Bibliothek aus nichtzufälligen Zielnucleinsäurevarianten zu bilden.

5. Verfahren nach Anspruch 1, wobei die Bibliothek eine Bibliothek aus Genen oder eine Bibliothek aus Stoffwechselwegen ist; wobei jede partielle doppelsträngige Nucleinsäure vorzugsweise eine Größe aufweist, die im Bereich von etwa 20 Basenpaaren bis etwa 200 Basenpaaren oder von etwa 500 Basenpaaren bis etwa 3000 Basenpaaren liegt.

6. Verfahren nach Anspruch 1, wobei jede partielle doppelsträngige Nucleinsäure aus einem Operon, umfassend eine Promotorsequenz, einer Ribosomenbindungsstellensequenz und einem Gen oder Satz von Genen und einer beliebigen Kombination daraus ausgewählt ist, wobei die Bibliothek vorzugsweise eine Bibliothek aus Operons, die Promotoren mit verschiedenen Stärken umfassen, oder eine Bibliothek aus Operons ist, die Ribosomenbindungsstellen mit verschiedenen Stärken umfassen.

7. Verfahren nach Anspruch 1, wobei das Verfahren weiters vor Schritt (a) Folgendes umfasst:

(i) Identifizieren einer Zielnucleinsäure;

(ii) Identifizieren einer ersten Region in der Zielnucleinsäure, wobei die erste Region eine Nucleinsäuresequenzvariante umfasst;

(iii) Identifizieren einer zweiten Region in der Zielnucleinsäure, wobei die zweite Region eine invariante Sequenz umfasst; und

(iv) Parsen der Zielnucleinsäure in zumindest einer ersten Vielzahl von Oligonucleotiden, umfassend die Nucleinsäuresequenzvariante, und zumindest einer zweiten Vielzahl von Oligonucleotiden, umfassend die invariante Nucleinsäuresequenz, wobei jedes aus der ersten Vielzahl von Oligonucleotiden eine Sequenz aufweist, die in der ersten Vielzahl von Oligonucleotiden einzigartig ist.

8. Verfahren nach Anspruch 7, wobei die Zielnucleinsäure für ein Polypeptid kodiert, das eine oder mehrere Domänen aufweist; wobei die erste Vielzahl von Oligonucleotiden vorzugsweise eine Deletion oder Insertion von Nucleinsäuresequenzen, die für zumindest einen Teil der einen oder der mehreren Domänen kodiert, oder eine Kombination daraus umfasst, wobei die Deletion oder die Insertion vorzugsweise ein Vielfaches von 3 Nucleotiden, noch bevorzugter fünf oder weniger Vielfache von 3 Nucleotiden oder bis zu 12 Vielfache von 3 Nucleotiden, ist.

9. Verfahren nach Anspruch 7, wobei die Zielnucleinsäure eine oder mehrere konstante Regionen und/oder eine oder mehrere variable Regionen umfasst.

10. Verfahren nach Anspruch 7, wobei die Bibliothek unter Verwendung eines Polymerase-basierten Ansatzes, eines Ligase-basierten Ansatzes oder einer Kombination daraus assembliert wird.

**11.** Verfahren nach Anspruch 7, wobei die Zielnucleinsäure ein Gen oder ein Satz von Genen ist, wobei die Nucleinsäurebibliothek vorzugsweise eine Deletion, eine Insertion oder eine Kombination daraus in der nichtkodierenden Sequenz des Gens oder des Satzes von Genen umfasst.

**12.** Verfahren nach Anspruch 1, wobei das Verfahren weiters vor Schritt (a) Folgendes umfasst:

(i) Auswählen einer Zielnucleinsäuresequenz;
(ii) Auswählen von zumindest einer zu deletierenden oder zu insertierenden Nucleinsäuresequenz an einer oder mehreren ausgewählten Positionen; und
(iii) Konstruieren eines ersten Satzes von Oligonucleotiden mit Sequenzvarianten an den ausgewählten Positionen und zumindest eines zweiten Satzes von Oligonucleotiden, umfassend eine invariante Sequenz, wobei jedes aus dem ersten Satz von Oligonucleotiden eine Sequenz aufweist, die in dem ersten Satz von Oligonucleotiden einzigartig ist.

**13.** Verfahren nach Anspruch 12, wobei die ausgewählten Positionen ein Nucleotid, ein Codon, eine Sequenz von Nucleotiden oder eine Kombination daraus umfassen.

**14.** Verfahren nach Anspruch 12, wobei in dem Schritt des Auswählens die zu deletierende oder zu insertierende Nucleinsäuresequenz ein Vielfaches von 3 Nucleotiden, vorzugsweise fünf oder weniger Vielfache von 3 Nucleotiden oder bis zu 12 Vielfache von 3 Nucleotiden, ist.

**15.** Verfahren nach Anspruch 12, wobei die Zielnucleinsäure ein Gen oder ein Satz von Genen ist, wobei die Nucleinsäurebibliothek vorzugsweise eine Deletion, eine Insertion oder eine Kombination daraus in der nichtkodierenden Sequenz des Gens oder des Satzes von Genen umfasst.

**Revendications**

**1.** Procédé pour générer une banque d'acides nucléiques comprenant une pluralité d'acides nucléiques cibles variants non aléatoires, uniques et prédéterminés, le procédé comprenant :

(a) la mise à disposition d'une première pluralité d'acides nucléiques double brin partiels dans un premier volume, dans lequel chacun de la première pluralité d'acides nucléiques double brin partiels est une séquence variante prédéterminée,
dans lequel un premier brin de chaque acide nucléique double brin partiel dans la première pluralité comporte des porte-à-faux simple brin identiques, et un second brin de chaque acide nucléique double brin partiel dans la première pluralité comporte des porte-à-faux simple brin identiques,
dans lequel chacun de la première pluralité d'acides nucléiques double brin partiels est une séquence variante prédéterminée qui est unique dans la première pluralité d'acides nucléiques double brin partiels ;
(b) la mise à disposition d'une deuxième pluralité d'acides nucléiques double brin partiels dans un deuxième volume,
dans lequel chacun de la deuxième pluralité d'acides nucléiques double brin partiels est une séquence invariante prédéterminée,
dans lequel un premier brin de chaque acide nucléique double brin partiel dans la deuxième pluralité comporte des porte-à-faux simple brin identiques et dans lequel un second brin de chaque acide nucléique double brin partiel dans la deuxième pluralité comporte des porte-à-faux simple brin identiques,
dans lequel le porte-à-faux simple brin du premier brin ou du second brin de chaque acide nucléique double brin partiel dans la deuxième pluralité est complémentaire au porte-à-faux simple brin du premier brin ou du second brin de chaque acide nucléique double brin partiel dans la première pluralité, et
dans lequel chacun de la deuxième pluralité d'acides nucléiques double brin partiels est la même séquence invariante prédéterminée ; et
(c) l'assemblage de la banque d'acides nucléiques en mélangeant la première pluralité d'acides nucléiques double brin partiels avec la deuxième pluralité d'acides nucléiques double brin partiels dans des conditions pour hybrider les porte-à-faux complémentaires afin de former la banque d'acides nucléiques cibles variants non aléatoires, uniques et prédéterminés.

**2.** Procédé selon la revendication 1 dans lequel, à l'étape d'assemblage, les porte-à-faux complémentaires s'hybrident pour former des jonctions sans brèches et sont ligaturés, dans lequel de préférence l'étape d'assemblage est réalisée dans un seul volume de réaction.

3. Procédé selon la revendication 1, dans lequel l'étape consistant à mettre à disposition la première et la deuxième pluralité d'acides nucléiques double brin partiels comprend :

(i) la mise à disposition d'une première pluralité d'ensembles d'acides nucléiques double brin aux extrémités franches dans le premier volume,

dans laquelle un premier acide nucléique d'un premier ensemble d'acides nucléiques double brin aux extrémités franches possède une séquence qui est décalée de n bases par rapport à un second acide nucléique du premier ensemble d'acides nucléiques double brin aux extrémités franches, et dans laquelle chaque acide nucléique double brin dans chaque ensemble d'acides nucléiques double brin aux extrémités franches est unique dans l'ensemble ;

(ii) la mise à disposition d'une deuxième pluralité d'ensembles d'acides nucléiques double brin aux extrémités franches dans le deuxième volume, dans laquelle un premier acide nucléique d'un second ensemble d'acides nucléiques double brin aux extrémités franches possède une séquence qui est décalée de n bases par rapport à un second acide nucléique du second ensemble d'acides nucléiques double brin aux extrémités franches ; (iii) la fusion de la première pluralité d'ensembles d'acides nucléiques double brin aux extrémités franches dans le premier volume en formant ainsi des acides nucléiques simple brin dans le premier volume et la fusion de la deuxième pluralité d'ensembles d'acides nucléiques double brin aux extrémités franches dans le deuxième volume en formant ainsi des acides nucléiques simple brin dans le deuxième volume ; et (iv) l'annelage des oligonucléotides simple brin pour former la première pluralité d'oligonucléotides double brin partiels dans le premier volume et la deuxième pluralité d'oligonucléotides double brin partiels dans le deuxième volume ; où de préférence n est 2, 3, 4, 5, 6, 7, ou 8 bases.

4. Procédé selon l'une quelconque des revendications 1 à 3, comprenant en outre une troisième pluralité d'acides nucléiques double brin partiels, dans lequel chacun de la troisième pluralité d'acides nucléiques double brin partiels comporte des porte-à-faux simple brin identiques, dans lequel chacun de la troisième pluralité d'acides nucléiques double brin partiels est unique dans la troisième pluralité d'acides nucléiques double brin partiels ; dans lequel de préférence le procédé comprend en outre l'assemblage de la banque d'acides nucléiques variants en mélangeant la première, la deuxième et la troisième pluralités d'acides nucléiques double brin partiels dans des conditions pour hybrider les porte-à-faux complémentaires afin de former la banque d'acides nucléiques cibles variants non aléatoires.

5. Procédé selon la revendication 1, dans lequel la banque est une banque de gènes ou une banque de voies métaboliques ; dans lequel de préférence chaque acide nucléique double brin partiel possède une taille comprise entre environ 20 paires de bases et environ 200 paires de bases ou entre environ 500 paires de bases et environ 3 000 paires de bases.

6. Procédé selon la revendication 1, dans lequel chaque acide nucléique double brin partiel est sélectionné parmi un opéron comprenant une séquence de promoteur, une séquence de site de liaison aux ribosomes et un gène ou un ensemble de gènes et une quelconque combinaison de ceux-ci, dans lequel de préférence la banque est une banque d'opérons comprenant des promoteurs possédant différentes puissances ou une banque d'opérons comprenant des sites de liaison aux ribosomes possédant différentes puissances.

7. Procédé selon la revendication 1, le procédé comprenant en outre, avant l'étape (a) :

(i) l'identification d'un acide nucléique cible ;
(ii) l'identification, dans l'acide nucléique cible, d'une première région, où la première région comprend une séquence d'acide nucléique variante ;
(iii) l'identification, dans l'acide nucléique cible, d'une deuxième région, où la deuxième région comprend une séquence invariante ; et
(iv) l'analyse de l'acide nucléique cible dans au moins une première pluralité d'oligonucléotides comprenant la séquence d'acide nucléique variante et au moins une deuxième pluralité d'oligonucléotides comprenant la séquence d'acide nucléique invariante, où chacun de la première pluralité d'oligonucléotides possède une séquence qui est unique dans la première pluralité d'oligonucléotides.

**8.** Procédé selon la revendication 7, dans lequel l'acide nucléique cible code pour un polypeptide comportant un ou plusieurs domaines ; dans lequel de préférence la première pluralité d'oligonucléotides comprend une délétion ou une insertion de séquences d'acide nucléique codant pour au moins une partie du un ou plusieurs domaines, ou une combinaison de ceux-ci ; dans lequel de préférence la délétion ou l'insertion est un multiple de 3 nucléotides, de manière davantage préférée cinq multiples ou moins de 3 nucléotides ou jusqu'à 12 multiples de 3 nucléotides.

**9.** Procédé selon la revendication 7, dans lequel l'acide nucléique cible comprend une ou plusieurs régions constantes et/ou une ou plusieurs régions variables.

**10.** Procédé selon la revendication 7, dans lequel la banque est assemblée en utilisant une approche basée sur une polymérase, une approche basée sur une ligase, ou une combinaison de celles-ci.

**11.** Procédé selon la revendication 7, dans lequel l'acide nucléique cible est un gène ou un ensemble de gènes, dans lequel de préférence la banque d'acides nucléiques comprend une délétion, une insertion ou une combinaison de celles-ci dans la région non codante du gène ou de l'ensemble de gènes.

**12.** Procédé selon la revendication 1, le procédé comprenant en outre, avant l'étape (a) :

(i) la sélection d'une séquence d'acide nucléique cible ;
(ii) la sélection d'au moins une séquence d'acide nucléique devant être supprimée ou insérée à une ou plusieurs positions sélectionnées ; et
(iii) la conception d'un premier ensemble d'oligonucléotides possédant des séquences variantes aux positions sélectionnées et d'au moins un deuxième ensemble d'oligonucléotides comprenant une séquence invariante, dans lequel chacun du premier ensemble d'oligonucléotides possède une séquence qui est unique dans le premier ensemble d'oligonucléotides.

**13.** Procédé selon la revendication 12, dans lequel les positions sélectionnées comprennent un nucléotide, un codon, une séquence de nucléotides ou une combinaison de ceux-ci.

**14.** Procédé selon la revendication 12 dans lequel, à l'étape de sélection, la séquence d'acide nucléique devant être supprimée ou insérée est un multiple de 3 nucléotides, de préférence cinq multiples ou moins de 3 nucléotides ou jusqu'à 12 multiples de 3 nucléotides.

**15.** Procédé selon la revendication 12, dans lequel l'acide nucléique cible est un gène ou un ensemble de gènes, dans lequel de préférence la banque d'acides nucléiques comprend une délétion, une insertion ou une combinaison de celles-ci dans la séquence non codante du gène ou de l'ensemble de gènes.

FIGURE 1A

FIGURE 1B

EP 3 074 554 B1

A₁      3′      B₁      3′

```
tccgatttacgggtagccgctgggttta
||||||||||||||||||||||||||||
agaacaggctaaatgcccatcggcgacc
```

5′      5′

FIGURE 2A

A₂      3′      B₁      3′

```
tccgatctacgggtagccgctgggttta
||||||||||||||||||||||||||||
agaacaggctagatgcccatcggcgacc
```

5′      5′

FIGURE 2B

FIGURE 3A

FIGURE 3B

FIGURE 3C

38

FIGURE 4A

FIGURE 4B

# Figure 5

## Codon level insertion and deletion:

Oligo 1 ATCGCTCAGGATTTCTTGGAAGAAGAAGCTAAGCACGCTGTTCAA---ATGTTGTTGCCAGAAAACGTTA

Oligo 1a ATCGCTCAGGAT---TTGGAACCGGAAGCTAAGCACGCTGTTCAA---ATGTTGTTGCCAGAAAACGTTA
(deletion)

Oligo 1b ATCGCTCAGGATCTGTTGGAACCGGAAGCTAAGCACGCTGTTCAACCGATGTTGTTGCCAGAAAACGTTA
(insertion)

## Nucleotide level insertion and deletion:

Oligo 2  GACTCTAAGGAAGACTCTTTGATCGCTACCGGT-TCTCACCACCACCACCACCACCACCAGTCTGGTGAC

Oligo 2a GACTCTAAGGAAGACTCTTTGATCGCTACCGGT-TCTCACCACCACCACCACCACCAC-AGTCTGGTGAC
(deletion)

Oligo 2b GACTCTAAGGAAGACTCTTTGATCGCTACCGGTATCTCACCACCACCACCACCACCACCAGTCTGGTGAC
(insertion)

## Multiple nucleotide insertion and deletion:

Oligo 3   ACCAATTGTCTCACTACAGACACGGTACCCTGACAGAAGATACAA------------TTGTTGTTCTT

Oligo 3a ACCAATTGTCTCACTAC................CTGACAGAAGATACAA------------TTGTTGTTCTT
(deletion)

Oligo 3b ACCAATTGTCTCACTACAGACACGGTACCCTGACAGAAGATACAACATGACCGAAGCTTGTTGTTCTT
(insertion)

## Variant 1

ATCGCTCAGGATTTCTTGGAAGAAGAAGCTAAGCACGCTGTTCAA
---ATGTTGTTGCCAGAAAACGTTAGACTCTAAGGAAGACTCTTT
GATCGCTACCGGT-TCTCACCACCACCACCACCACCACCAGTCTG
GTGACACCAATTGTCTCACTAC................CTGACAGAAG
ATACAA------------TTGTTGTTCTT

## Variant 2

ATCGCTCAGGAT---TTGGAACCGGAAGCTAAGCACGCTGTTCAA---
ATGTTGTTGCCAGAAAACGTTA GACTCTAAGGAAGACTCTTTGATC
GCTACCGGT-TCTCACCACCACCACCACCACCAC-AGTCTGGTGAC
ACCAATTGTCTCACTAC................CTGACAGAAGATA
CAA------------TTGTTGTTCTT

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5942609 A
- US 200955267 W
- US 2010055298 W
- US 5770358 A
- US 5639603 A
- US 5541061 A
- US 61909537 B

**Non-patent literature cited in the description**

- **GRABAR et al.** *Analyt. Chem.,* 1995, vol. 67, 73-743
- **BETHELL et al.** *J. Electroanal. Chem.,* 1996, vol. 409, 137
- **BAR et al.** *Langmuir,* 1996, vol. 12, 1172
- **COLVIN et al.** *J. Am. Chem. Soc.,* 1992, vol. 114, 5221